# EUROPEAN PATENT APPLICATION

(11) **EP 4 570 306 A1**
(43) Date of publication of application: **18.06.2025**
(21) Application number: 24216439.0
(22) Date of filing: 29.11.2024
(51) Int. Cl.: A61N 1/372, A61B 17/34, A61M 25/00, A61F 2/95, A61N 1/375, A61N 1/05, A61B 17/00, A61M 25/01

(54) **CONTROL HANDLE WITH A LOCKING MECHANISM FOR CONTROLLING FUNCTIONS OF A DELIVERY SYSTEM FOR IMPLANTING A MEDICAL DEVICE**

(30) Priority: 15.12.2023 US 202363610650 P; 09.01.2024 EP 24150970
(71) Applicant: BIOTRONIK SE & Co. KG, 12359 Berlin (DE)
(72) Inventor: DEVICH, Nicholas, Tualatin, WA 97062 (US); SIEGEL, Philipp Konstantin, 10318 Berlin (DE); AUSTIN, Eric, Portand, WA 97221 (US)
(74) Representative: Biotronik Corporate Services SE

(57) **Abstract**

A control handle (1) for controlling functions of a delivery system (3) for implanting a medical device is described. The delivery system comprises an elongate delivery catheter (5) and a mandrel (7) being displaceable longitudinally relative to the delivery catheter. The control handle (1) comprises a locking mechanism (19) and a handle housing (9). The locking mechanism is configured for being switchable between a locked configuration and an unlocked configuration such that, in the locked configuration, the mandrel is fixed relative to the locking mechanism such that the mandrel is not displaceable relative to the locking mechanism beyond a predetermined abutment position due to a mechanical mandrel abutment blockage, whereas, in the unlocked configuration, the mandrel is movable in the longitudinal direction relative to the locking mechanism beyond the predetermined abutment position due to the mandrel abutment blockage being released.

## Description

The present invention relates to a control handle for controlling functions of a delivery system for implanting a medical device. Furthermore, the present invention relates to a delivery system comprising such control handle.

There are various medical devices (also referred to as implantable medical devices - IMD) which have to be implanted into a patient at locations inside the patient's body. For example, leadless pacemakers (ILP, sometimes also referred to as intracardiac pacemakers) are to be implanted directly into a patient's heart.

Generally, an IMD and an ILP in particular is introduced into the patient's body, forwarded to an intended implantation location and fixed at such intended location using a specific delivery system. Such delivery system typically comprises an elongate delivery catheter and a mandrel. Therein, the catheter may be steerable and/or guidable such that a distal end of the catheter may be displaced throughout the patient's body, i.e. for example along vessels of the patient, until reaching the intended implantation location, i.e. for example the patient's heart. The mandrel is generally displaceable longitudinally relative to the delivery catheter such as to induce or control functionalities of the delivery system. For example, the mandrel may be displaced in order to expose the IMD from a protector sheath, execute a tug test and/or finally release the IMD from the delivery catheter (as will be explained in further detail below).

Functions of the delivery system are generally controlled using a control handle. Such control handle is typically arranged at a proximal end of the delivery system such that for example a surgeon may use the control handle for forwarding and/or steering the delivery catheter and/or for suitably displacing the mandrel.

Examples of delivery systems and catheter devices for implanting a medical device are for example described in the applicant's prior applications WO 2020/043481 A1 and WO 2020/187663 A1. It is to be noted that the control handle described herein may be used or adapted for controlling functions of such prior art delivery systems and catheter devices and that characteristics and/or functionalities described in the prior applications may be implemented in the delivery system and its control handle as described in the present application. Accordingly, the content of the cited prior applications shall be incorporated herein in its entirety by reference.

There may be a need for a control handle for controlling functions of a delivery system for implanting a medical device, the control handle providing improved ergonomics and/or enabling simplified or more precise handling of a delivery system. Furthermore, there may be a need for a delivery system comprising such control handle.

Such needs may be met with the subject-matter of the independent claims. Advantageous embodiments are defined in the dependent claims as well as in the corresponding specification and figures.

According to a first aspect of the present invention, a control handle for controlling functions of a delivery system for implanting a medical device is proposed. Therein, the delivery system comprises an elongate delivery catheter and a mandrel being displaceable longitudinally relative to the delivery catheter. The control handle comprises a locking mechanism and a handle housing. Therein, the locking mechanism is configured for being switchable between a locked configuration and an unlocked configuration such that, in the locked configuration, the mandrel is fixed relative to the locking mechanism such that the mandrel is not displaceable relative to the locking mechanism beyond a predetermined abutment position due to a mechanical mandrel abutment blockage, whereas, in the unlocked configuration, the mandrel is movable in the longitudinal direction relative to the locking mechanism beyond the predetermined abutment position due to the mandrel abutment blockage being released.

According to a second aspect of the invention, a delivery system is proposed, the delivery system comprising a control handle according to an embodiment of the first aspect of the invention, an elongate delivery catheter mechanically connected to the control handle and a mandrel being controlled by the control handle such as to be displaceable longitudinally relative to the delivery catheter.

Ideas underlying embodiments of the present invention may be interpreted as being based, inter alia, on the following observations and recognitions.

Briefly summarised in a non-limiting manner, embodiments of the present invention relate to a control handle which may be used together with other components of a delivery system for controlling functions of the delivery system during a procedure for implanting an IMD such as a leadless pacemaker. Therein, the control handle comprises a locking mechanism which is arranged at or in a handle housing. During surgery procedures, the locking mechanism may be actuated for example by a user of the delivery system such as a surgeon. Particularly, the locking mechanism may be actuated such as to be switched between a first configuration referred to herein as locked configuration and a second configuration referred to herein as unlocked configuration. In the locked configuration, the locking mechanism is configured for blocking any displacement of the mandrel relative to the locking mechanism beyond a predetermined abutment position due to a mechanical blockage referred to herein as mandrel abutment blockage. In the unlocked configuration, the mechanical mandrel abutment blockage is released, thereby allowing the mandrel to be displaced beyond the abutment position.

The abutment position may be determined by the length of various portions of the mandrel, the portions having different diameters, as described in further detail below. Particularly, the abutment position may be set such that, unless the mandrel is displaced beyond the abutment position, a locking element provided at the distal end of the mandrel and being configured for releasably fixing the IMD to the mandrel may not be located at a position at which the locking element is opened and thereby enable is irreversibly releasing the IMD. Accordingly, as long as the locking mechanism at the control handle is in its locked configuration, the locking element at the distal end of the mandrel may not be released. Thus, unless the locking mechanism is intentionally actuated by switching it to its unlocked configuration, no unintended releasing of the IMD from the distal end of the mandrel may occur.

Within the frame of this application mandrel is understood as a wire inserted into the delivery catheter or into a sheath of a delivery catheter. The mandrel is stiffer than the delivery catheter or the sheath into which the mandrel is inserted into. The mandrel may be a made of metal. The mandrel may be a solid body.

The locking mechanism may be incorporated into the control handle together with other features or elements for controlling other functions of the delivery system such as for example for controlling a deployment of the IMD and/or a tether mode. For example, the locking mechanism may be configured for being actuated by a rotation motion of one of its components.

All these characteristics support improving ergonomics of the control handle. Particularly, unintended releasing of the IMD by displacing the mandrel beyond the predetermined abutment position may reliably be prevented until the locking mechanism is switched to the unlocked configuration and such switching may be accomplished by a surgeon using a simple actuation of the locking mechanism at the control handle.

In the following, characteristics of embodiments of the present invention will be described in more detail.

The control handle is generally configured for controlling two or more functions of a delivery system upon implanting an IMD, in particular an ILP.

The delivery system comprises a delivery catheter and a mandrel. The catheter is elongate, i.e. has substantially larger dimensions in a longitudinal direction than in a lateral direction. For example, the catheter may have a length of more than 10 cm, typically between 50 cm and 200 cm, while having a diameter of less than 5 cm, typically between 0.5 and 2 cm. The catheter is bendable in its lateral direction such that it may be steered and/or guided along a curved path e.g. throughout vessels of a patient. Generally, the catheter has an inner lumen through which the mandrel extends. The mandrel typically is a wire or rope. Therein, the mandrel is generally bendable in a lateral direction but is substantially inelastic in the longitudinal direction.

The locking mechanism and the handle housing are components of the control handle. The control handle may comprise further components such as e.g. a steering mechanism for steering the delivery catheter or a slider mechanism for displacing the mandrel between an undeployed configuration and the deployed configuration and, optionally, for enabling a tether mode of the delivery system. All such components may be made with a material suitable for medical applications such as to be for example suitably disinfectable. For example, some or all of the components may be made with plastic material and, optionally, may be injection moulded components. Alternatively or additionally, at least some of the components may be made with a metal such as stainless steel or nitinol.

The locking mechanism may be displaced relative to the handle housing. Particularly, for example at the beginning of a surgery procedure, the locking mechanism may be arranged in the locked configuration. In such locked configuration, the locking mechanism prevents motions of the mandrel resulting in releasing the IMD, in particular an ILP, from the delivery system. Later during the surgery procedure, for example after having positioned the IMD at a correct implantation site and, optionally, after having conducted successful tests such as a tug test and/or a test of functionalities of the IMD, the surgeon may decide that the IMD is correctly implanted and anchored and may therefore switch the locking mechanism to its unlocked configuration, thereby intentionally enabling the displacement of the mandrel to a position at which it enables releasing the IMD from the delivery system.

According to an embodiment, the locking mechanism is configured for being switched between the locked configuration and the unlocked configuration by rotating an unlock actuation member relative to the handle housing.

In other words, the locking mechanism comprises, inter-alia, an unlock actuation member which may be turned between different states, thereby switching the locking mechanism between the locked configuration and the unlocked configuration. On the one hand, such rotational actuation of the switching procedure may be easily performed by a surgeon and may be ergonomically beneficial while, on the other hand, reliably preventing any unintended actuation of the switching procedure.

According to a further specified embodiment, a rotation axis of the unlock actuation member is aligned with a longitudinal axis of the control handle.

Expressed differently, the unlock actuation member of the locking mechanism may be actuated by turning it around the longitudinal axis of the control handle. Again, such rotational actuation of the switching procedure may be ergonomically beneficial and may be easily technically implemented.

Furthermore, according to an embodiment, the locking mechanism may be configured for being switched between the locked configuration and the unlocked configuration by screwing the unlock actuation member in a clockwise direction.

While, in principle, the locking mechanism could be technically implemented for actuation in both, the counter clockwise direction or the clockwise direction, it has been found that by configuring the locking mechanism to be actuated into the unlocked configuration by screwing the unlock actuation member in the clockwise direction, unintended actuation of the locking mechanism may be prevented more reliably.

According to an embodiment, the locking mechanism comprises a through-passage for accommodating the mandrel, with lateral limiting elements of the locking element enclosing the through-passage from opposite sides. Therein, the locking mechanism is configured such that, in the locked configuration, the lateral limiting elements are pushed towards each other more than in the unlocked configuration such that, in the locked configuration, lateral dimensions of the through-passage are smaller than in the unlocked configuration.

In other words, the locking mechanism may comprise a through-passage such as a through-hole or a groove through which the mandrel may extend. Such a through-passage may be formed by portions or components of the locking element referred to herein as lateral limiting elements. The lateral limiting elements enclose the through-passage from opposite sides such that the actual cross-sectional dimensions of the through-passage are determined by the actual location of the lateral limiting elements. The lateral limiting elements may be configured for being elastically biased towards a first configuration, also referred to herein as unloaded configuration. In such unloaded configuration, no or only weak lateral compression forces act onto the lateral limiting elements such that the through-passage may have a relatively large cross-sectional dimension or diameter. Such unloaded configuration corresponds to the unlocked configuration of the locking mechanism. However, upon inducing stronger lateral compression forces onto the lateral limiting elements, the lateral limiting elements are pushed towards each other into a second configuration, also referred to herein as loaded configuration. In reaction to such increased lateral compression forces, the cross-sectional dimension or diameter of the through-passage is reduced. Such loaded configuration of the lateral limiting elements corresponds to the locked configuration of the locking mechanism.

According to a further specified embodiment, the locking mechanism comprises a compression element which, upon rotating an unlock actuation member from the locked configuration to the unlocked configuration, releases compression forces radially acting onto the lateral limiting elements.

Expressed differently, by rotating the unlock actuation member from the locked configuration to the unlocked configuration, the lateral compression forces initially reducing the cross-sectional dimensions of the through-passage limited by the lateral limiting elements may be reduced to an extend such that the cross-sectional dimensions of the through-passage may increase towards the unloaded configuration. The compression element may be the unlock actuation member itself or may be a separate component cooperating with the unlock actuation member.

According to a further specified embodiment, the lateral limiting elements are formed by an inner member enclosing the through-passage and having longitudinal slits extending between elongate portions of the inner member and furthermore having a conical outer shape at its cantilever end. Furthermore, the compression element is formed by an outer member enclosing the inner member and having a conical inner shape at an inner surface opposing the cantilever end of the inner member.

In other words, the locking mechanism may include an inner member and an outer member. The inner member may include several elongate portions extending in the longitudinal direction and parallel to each other such as to enclose the through-passage between elongate portions opposing to each other relative to the longitudinal middle axis of the through-passage. The elongate portions may be held at one end and may be elastically deflectable at an opposite cantilever end. The elongate portions may have a shape such as to be conical at its outer surface at the cantilever end. The outer member is configured for cooperating with the inner member such as to exert varying compression forces onto the elongate portions of the inner member at or close to their cantilever end, the compression forces varying in dependence of an actuation status of the locking mechanism. Particularly, the outer member encloses the inner member and has a shape such that an inner surface of the outer member is conical at least in an area opposing the cantilever end of the inner member.

The outer member and the inner member may be configured such that they are displaced relative to each other in the longitudinal direction upon the locking mechanism being actuated between the locked configuration and the unlocked configuration. For example, when actuating from the locked configuration to the unlocked configuration, the outer member and the inner member may be displaced such that the conical outer surface of the inner member moves away from the opposing conical inner surface of the outer member. As a result of such motion, compression forces exerted by the outer member onto the cantilever end of the inner member may be reduced such that, due to the elongate portions of the inner member being elastically biased in a radial outward direction, the cross-sectional dimensions of the through-passage enclosed by the inner member is enlarged.

According to a further specified embodiment, the mandrel comprises a tether hypotube portion and a release hypotube portion being arranged further proximal to the tether hypotube portion. The tether hypotube portion has a first cross-sectional dimension and the release hypotube portion has a second cross-sectional dimension being larger than the first cross-sectional dimension. Therein, upon the locking mechanism being in the locked configuration, the through-passage of the locking mechanism has a cross-sectional dimension being smaller than the second cross-sectional dimension of the release hypotube portion, whereas, upon the locking mechanism being in the unlocked configuration, the through-passage of the locking mechanism has a cross-sectional dimension being larger than the second cross-sectional dimension of the release hypotube portion.

In such embodiment, the mandrel typically comprises at least two portions referred to herein as tether hypotube portion and release hypotube portion. As these portions have different cross-sectional dimensions, the smaller tether hypotube portion may extend through the through-passage of the locking mechanism upon the locking mechanism being in its locked configuration and therefore having a smaller cross-sectional dimension, whereas the larger release hypotube portion may only extend through the through-passage of the locking mechanism when the locking mechanism is in its unlocked configuration and therefore has a larger cross-sectional dimension.

Accordingly, as long as the locking mechanism is in its locked configuration, the mandrel may only be longitudinally displaced as long as its tether hypotube portion extends through the through-passage whereas any further longitudinal displacement is blocked upon the mandrel abutting to the through-passage with its large-diameter release hypotube portion. Such mandrel abutment blockage may be released only by actuating the locking mechanism into its unlocked configuration, thereby enlarging the cross-sectional dimensions of the through-passage and thus allowing the release hypotube portion to be inserted into the through-passage, thereby enabling further longitudinal distal displacement of the mandrel to a position in which the IMD held at its distal end may be released.

According to an embodiment, the control handle further comprises a haptic locker feedback mechanism. The haptic locker feedback mechanism is configured for generating a haptic locker feedback to a user of the handle upon the locking mechanism being displaced relative to the handle housing beyond at least one locker feedback position upon switching from the locked configuration to the unlocked configuration.

Due to the haptic locker feedback generated by such haptic locker feedback mechanism, a user of the control handle may be informed about specific configurations occurring upon operating the control handle. For example, the locker feedback may be given upon specific configurations being reached during switching the locker mechanism from the locked configuration to the unlocked configuration. As such locker feedback is given in a haptic manner, the user does not need to for example visually observe the control handle. Giving such haptic locker feedback may improve ergonomically and reliably handling the delivery system by a user.

According to a further specified embodiment, the haptic locker feedback mechanism comprises at least one groove and at least one spring plunger, the groove being arranged at one of the handle housing and the locking mechanism and the spring plunger being arranged at the other one of the handle housing and the locking mechanism. Therein, the groove and the spring plunger may be configured such as to induced a retracting force onto the locking mechanism upon a user rotating the locking mechanism beyond the at least one locker feedback position.

The groove and the spring plunger may be configured such as to cooperate with each other upon being aligned with each other such as to generate the retracting force. For example, the spring plunger may be elastically biased towards the groove such that, when the spring plunger and the groove are aligned, the spring plunger may "snap-in" into the groove. For example, the spring plunger may protrude from a surface of the locking mechanism towards an opposing surface of the handle housing and the groove may form a depression extending along the opposing surface of the handle housing, or vice versa.

By inducing the retracting force onto the locking mechanism upon passing the locker feedback position, the arrangement including the groove and the spring plunger may generate the haptic locker feedback to the user relating to a specific configuration of the delivery system to be indicated to the user. The retracting force may decelerate the rotational displacement of the locking mechanism and/or may require the user to temporarily increase the force acting onto the locking mechanism in order to rotate the locking mechanism beyond the locker feedback position. Accordingly, a surgeon may haptically feel for example when he activates the locking mechanism from its locked configuration to its unlocked configuration, thereby for example enabling a release mode of the delivery system in which the IMD may be released from the delivery system.

The delivery system according to the second aspect of the invention comprises a control handle according to an embodiment of the first aspect of the invention. Furthermore, the delivery system comprises an elongate delivery catheter mechanically connected to the control handle and a mandrel being controlled by the control handle such as to be displaceable longitudinally relative to the delivery catheter. Therein, the control handle may be configured and may cooperate with the delivery catheter and the mandrel as described further above and further below. Furthermore, the delivery catheter and the mandrel may be configured for implementing various functionalities.

Particularly, according to an embodiment, the delivery system may be configured such that, upon the mandrel being displaced relative to the delivery catheter by a deployment distance, the medical device, in particular an ILP, held at a distal end of the delivery catheter is deployed from a protector sheath of the delivery catheter. Furthermore, the delivery system may further comprise a tethering member being connected to a distal end of the mandrel and being connected to the medical device. The delivery system may then be configured such that, upon the mandrel being displaced relative to the delivery catheter by a tethering distance additionally to the deployment distance, the tethering member is displaced between a retracted position and an ejected position. Therein, in the retracted position, the tethering member pulls the medical device into fixation with an end cup at the distal end of the delivery catheter whereas, in the ejected position, the tethering member releases the medical device from the fixation with the end cup. Additionally, the delivery system is further configured such that, upon the mandrel being displaced relative to the delivery catheter by a release distance additionally to the tethering distance and the deployment distance, the tethering member is displaced between the ejected position and a release position, wherein, in the release position, the tethering member releases the medical device from any connection with the delivery system.

According to a further specified embodiment, the mandrel comprises a bare mandrel portion, a tether hypotube portion being arranged further proximal to the bare mandrel portion and a release hypotube portion being arranged further proximal to the tether hypotube portion. The bare mandrel portion has a first cross-sectional dimension, the tether hypotube portion has a second cross-sectional dimension being larger than the first cross-sectional dimension and the release hypotube portion has a third cross-sectional dimension being larger than the second cross-sectional dimension. Therein, the length of the bare mandrel portion corresponds to or is longer than the deployment distance, the length of the tether hypotube portion corresponds to or is longer than the tethering distance and the length of the release hypotube portion corresponds to or is longer than the release distance.

Additional to the characteristics described herein with reference to various embodiments of the invention, the control handle and the delivery system may be configured in accordance with various additional aspects and implementations. For example, such additional aspects and implementations are described in another patent application filed by the applicant contemporaneous with the present application and having the title "control handle with a slider mechanism for controlling functions of a delivery system for implanting a medical device", the content of which shall be incorporated herein in its entirety by reference. Examples of such additional aspects and implementations are described in the following paragraphs.

According to a first additional aspect, a control handle for controlling functions of a delivery system for implanting a medical device is proposed. Therein, the delivery system comprises an elongate delivery catheter and a mandrel being displaceable longitudinally relative to the delivery catheter. The control handle comprises a slider mechanism and a handle housing. The slider mechanism is configured for being displaceable in a longitudinal direction relative to the handle housing between a fully undeployed position and a fully deployed position such as to thereby displacing the mandrel relative to the delivery catheter by a deployment distance. The slider mechanism is further configured such that, in the fully undeployed position, the mandrel is fixed relative to the slider mechanism such that the mandrel is not displaceable relative to the slider mechanism beyond a predetermined stop position due to a mechanical mandrel motion blockage, whereas, in a tether position being beyond the fully deployed position, the mandrel is movable in the longitudinal direction relative to the slider mechanism beyond the predetermined stop position due to the mandrel motion blockage being released.

According to a second additional aspect, a delivery system is proposed, the delivery system comprising a control handle according to an implementation of the first additional aspect, an elongate delivery catheter mechanically connected to the control handle and a mandrel being controlled by the control handle such as to be displaceable longitudinally relative to the delivery catheter.

Ideas underlying implementations of such additional aspects may be interpreted as being based, inter alia, on the following observations and recognitions.

Briefly summarised in a non-limiting manner, implementations of such additional aspects relate to a control handle which may be used together with other components of a delivery system for controlling functions of the delivery system during a procedure for implanting an IMD such as a leadless pacemaker. Therein, the control handle comprises a slider mechanism which is arranged at or in a handle housing. The slider mechanism may be actuated for example by a user of the delivery system such as a surgeon. Particularly, the slider mechanism may be displaced in a longitudinal direction of the control handle between a first position, referred to herein as fully undeployed position, and a second position, referred to herein as fully deployed position. Upon displacing the slider mechanism between such longitudinally opposing extremal positions, the slider mechanism cooperates with the mandrel and displaces the mandrel relative to the delivery catheter by a predetermined distance referred to herein as deployment distance. Due to such displacement of the mandrel, the IMD being connected to the distal end of the mandrel may be displaced relative to a protector cup provided at a distal end of the delivery catheter such as to deploy the IMD from such protector cup.

Additional to implementing such deployment motion by displacing the mandrel, the slider mechanism is furthermore configured to automatically disable or enable an additional motion of the mandrel relative to the slider mechanism depending on the current position of the slider mechanism relative to the handle housing. Particularly, the slider mechanism is configured to establish a mechanical mandrel motion blockage when positioned in the fully undeployed position and to release such mechanical mandrel motion blockage when slid to the fully deployed position and then reaching a so-called tether position being beyond the fully deployed position. When the mandrel motion blockage is established, the slider mechanism cooperates with the mandrel such that the mandrel may not be displaced relative to the slider mechanism beyond a predetermined stop position. In the delivery system, such displacing the mandrel beyond the deployed position but not beyond the predetermined stop position may allow establishing a so-called tether configuration in which the IMD is released from a fixation with the catheter but is still connected to the catheter via a tether. The predetermined stop position may be set such that the mandrel motion blockage prevents displacing the mandrel beyond such tether configuration, i.e. for example to a release configuration in which the mandrel is ejected from the catheter to such distance such that the tether configuration is released and the mechanical connection between the IMD and the catheter is opened.

Accordingly, the slider mechanism in the control handle may be ergonomically and precisely actuated. Particularly, the slider mechanism may simplify a deployment procedure established with the delivery system and may then automatically switch from a deployment mode to a tether mode in which the IMD is fully deployed and ejected from the delivery catheter but is still tethered by the delivery system and therefore enables conducting tests such as a tug test and, if necessary e.g. due to such tug test being negative, recapturing the IMD.

In the following, characteristics of implementations of such additional aspects will be described in more detail.

The control handle is generally configured for controlling two or more functions of a delivery system upon implanting an IMD, in particular an ILP.

The delivery system comprises a delivery catheter and a mandrel. The catheter is elongate, i.e. has substantially larger dimensions in a longitudinal direction than in a lateral direction. For example, the catheter may have a length of more than 10 cm, typically between 50 cm and 200 cm, while having a diameter of less than 5 cm, typically between 0.5 and 2 cm. The catheter is bendable in its lateral direction such that it may be steered and/or guided along a curved path e.g. throughout vessels of a patient. Generally, the catheter has an inner lumen through which the mandrel extends. The mandrel typically is a wire or rope. Therein, the mandrel is generally bendable in a lateral direction but is substantially inelastic in the longitudinal direction.

The slider mechanism and the handle housing are components of the control handle. The control handle may comprise further components such as e.g. a steering mechanism for steering the delivery catheter or a locking mechanism for blocking any unintended mandrel motion resulting in finally releasing the IMD from the delivery system. All such components may be made with a material suitable for medical applications such as to be for example suitably disinfectable. For example, some or all of the components may be made with plastic material and, optionally, may be injection moulded components. Alternatively or additionally, at least some of the components may be made with a metal such as stainless steel or nitinol.

The slider mechanism may be displaced in a longitudinal direction relative to the handle housing. Particularly, for example at a beginning of a surgery procedure, the slider mechanism may be arranged in the fully undeployed position. Such fully undeployed position may be a position at which the slider mechanism is arranged at a maximum proximal location relative to the handle housing. When arranged in such fully undeployed position, the mandrel cooperating with the slider mechanism may be arranged such that the IMD being held at the distal end at the mandrel is arranged in an undeployed configuration in which it is accommodated within the delivery catheter, preferably within a protector cup of the delivery catheter. During the surgery procedure, the slider mechanism may then be displaced for example by a surgeon pushing the slider mechanism relative to the handle housing away from the fully undeployed position towards the fully deployed position. Upon reaching such fully deployed position, the mandrel is arranged in a deployed configuration in which the IMD is deployed, i.e. is released from the accommodation and/or fixation with the delivery catheter and may be e.g. anchored within tissue of the patient that an implantation site.

Additional to the motion between the fully undeployed position and the fully deployed position, the slider mechanism allows a further motion towards a tether position. Such tether position may be reached upon the slider mechanism being longitudinally displaced to the fully deployed position and may then actively or passively move further beyond such position. Upon being arranged at such tether position, the mandrel may not only be moved to the fully deployed configuration but may further be moved to a tether configuration in which the IMD at its distal end is in a tether mode, i.e. is loose with regards to the catheter. Preferably, the tether position is arranged at a position being distant in a lateral direction relative to the fully deployed position. In other words, while the slider mechanism may be moved in the longitudinal direction between the fully undeployed position and the fully deployed position, it may further move in a lateral direction crossing or preferably being orthogonal to such longitudinal direction in order to come to the tether position.

According to an implementation, the slider mechanism is configured such that, when the slider mechanism is displaced to the fully deployed position and then all forces applied by a user are released, the slider mechanism automatically displaces into the tether position, thereby releasing the mandrel motion blockage.

In other words, the slider mechanism may be configured such as, when the slider mechanism reaches the fully deployed position, it automatically moves further towards the tether position without requiring any further forces to be applied by for example a surgeon.

Accordingly, upon the delivery system reaching the deployed configuration and the surgeon e.g. releasing his actuation of the slider mechanism, the control handle automatically switches to a tether mode, in which the mandrel may be further displaced relative to the delivery catheter beyond the predetermined stop position. Thus, the mandrel motion blockage, which is active during an initial portion of a surgery sequence in which the IMD has first to be deployed, is automatically released upon the slider mechanism reaching the fully deployed position and then further moving to the tether position. Such automatic motion and release of the mandrel motion blockage may significantly simplify a handling of the delivery system.

According to an implementation, the slider mechanism comprises a slider body and a slider housing. Therein, the slider body is arranged at the slider housing in a manner such as to be elastically biased in a lateral direction away from the slider housing. Furthermore, the slider body and the slider housing are configured such that, as long as the slider mechanism is positioned at the fully undeployed position or somewhere between the fully undeployed and fully deployed position, the slider body is kept fixed to the slider housing in a pulled-down configuration, whereas, upon arriving at the fully deployed position, the fixation is released such that the slider body automatically displaces in a lateral direction away from the slider housing into a pulled-up configuration due to the elastic biasing, thereby releasing the mandrel motion blockage.

Expressed differently, the slider mechanism may comprise at least two sub-components which may move relative to each other. Particularly, the slider body and the slider housing may move relative to each other in the lateral direction, such lateral direction crossing the longitudinal direction of the control handle. Therein, the slider body may be elastically biased, i.e. there may be an elastic force acting onto the slider body, such that the slider body is elastically pre-tensioned in a lateral direction away from the slider housing. The elastic bias may be implemented using for example one or more springs or other elastic elements. Such elastic elements may be interposed between the slider housing and the slider body.

In order to implement a beneficial and, preferably, automatic functionality of the slider mechanism, the slider mechanism together with its slider body and slider housing are configured such that the slider body remains in the pulled-down configuration as long as the slider mechanism is somewhere between the fully undeployed position and the fully deployed position but, as soon as reaching the fully deployed position and, furthermore, the slider body not being actively pressed down to its pulled down configuration for example by the surgeon, the slider body automatically transfers to the pulled-up configuration. Therein, in the pulled-down configuration, the slider body is closer to a central axis of the control handle whereas, in the pulled-up configuration, the slider body is laterally displaced and therefore being further away from the central axis.

Accordingly, between the fully undeployed position and the fully deployed position, the slider mechanism has only a single degree of motion freedom and may only be slid in the longitudinal direction, whereas, upon having reached the fully deployed position, the slider mechanism with its slider body obtains a further degree of motion freedom in which the slider body may be displaced laterally from the pulled-down configuration to the pulled-up configuration.

Thus, a surgeon may easily push the slider mechanism from the initial fully undeployed position to the fully deployed position and then, upon the surgeon releasing a lateral pressure onto the slider mechanism for example by removing his thumb, the slider body may "pop-up" into the pulled-up configuration and may thereby release the mandrel motion blockage.

Having reached the pulled-up configuration, the mandrel may therefore be moved in the longitudinal direction relative to the slider mechanism beyond the predetermined stop position, thereby activating the tether mode of the delivery system. In other words, if the slider body is in the pulled-down configuration, any movement of the slider is transferred to a corresponding movement of the mandrel. If the slider body is in the pulled-up configuration, the mandrel can be moved independently from the slider.

According to an implementation, the slider body and the slider housing each comprise a through-hole extending in the longitudinal direction such that the mandrel extends longitudinally through both through-holes. Therein, the through-hole of the slider body comprises two sections with a first section having a smaller cross-sectional dimension than a second section such that, when the slider body is in the pulled-down configuration, the mandrel extends through the first section and when the slider body is in the pulled-up configuration, the mandrel extends through the second section.

In other words, the mandrel may extend along a straight line which goes through through-holes extending through both, the slider body and the slider housing. While the through-hole in the slider housing may have for example a circular cross section with a single diameter being larger than the mandrel, the through-hole in the slider body includes two sections each of which may have a circular cross section but the two sections having different diameters. The two sections laterally overlap such that they form a single through-hole having a contour with one smaller first section and one larger second section, similar to the contour of a keyhole. Therein, the first section of the through-hole of the slider body generally has smaller lateral dimensions than the through-hole of the slider housing, whereas the second section of the through-hole of the slider body generally has same or larger lateral dimensions than the through-hole of the slider housing.

As long as the slider body is in its pulled-down configuration, the first section of its through-hole having the smaller dimensions is aligned with the through-hole of the slider housing. Accordingly, a portion of the mandrel extending through both through holes in the slider body and the slider housing must have a smaller diameter than the smaller dimensions of the first section of the through-hole in the slider body in order to be able to be laterally displaced along the slider mechanism.

However, when the slider body is in its pulled-up configuration, the second section of its through-hole having the larger dimensions is aligned with the through-hole in the slider housing. Accordingly, the portion of the mandrel extending through the slider body and slider housing may have larger dimensions than in the first case, i.e. its diameter may be as large as the diameter of the through-hole through the slider housing and the second section of the through-hole through the slider body.

According to an implementation, the mandrel comprises a bare mandrel portion and a tether hypotube portion being arranged further proximal to the bare mandrel portion. The bare mandrel portion has a first cross-sectional dimension and the tether hypotube portion has a second cross-sectional dimension being larger than the first cross-sectional dimension. The first section of the through-hole of the slider body has a cross-sectional dimension being smaller than the second cross-sectional dimension of the tether hypotube portion, whereas the second section of the through-hole of the slider body has a cross-sectional dimension being larger than the second cross-sectional dimension of the tether hypotube portion.

In such implementation, the mandrel typically comprises at least two different portions referred to herein as bare mandrel portion and tether hypotube portion. As these portions have different cross-sectional dimensions, the smaller bare mandrel portion may extend through a through-hole having smaller cross-sectional dimensions whereas the larger tether hypotube portion may extend only through a through-hole having larger cross-sectional dimensions. Therein, the cross-sectional dimensions of the mandrel are set such that the tether hypotube portion may extend through the slider mechanism and may therefore be displaced in the longitudinal direction only, when the slider body is in its pulled-up configuration and therefore the mandrel extends along the larger second section of its through-hole.

According to an implementation, the control handle further comprises a haptic deployment feedback mechanism. The haptic deployment feedback mechanism is configured for generating a haptic deployment feedback to a user of the handle upon the slider mechanism being displaced beyond at least one predetermined deployment feedback position situated longitudinally between the fully undeployed position and the fully deployed position.

Due to the haptic deployment feedback generated by such haptic deployment feedback mechanism, a user of the control handle may be informed about specific configurations occurring upon operating the control handle. For example, a deployment feedback may be given upon specific configurations being reached during displacing the slider mechanism from the fully undeployed position to the fully deployed position. As such deployment feedback is given in a haptic manner, the user does not need to for example visually observe the handle. Giving such deployment haptic feedback may improve ergonomically and reliably handling the delivery system by a user.

According to an implementation, a predetermined first deployment feedback position corresponds to a position such that, upon the slider mechanism being between the fully undeployed position and the first deployment feedback position, the slider mechanism positions the mandrel into a sheathed configuration in which the mandrel and the delivery catheter are positioned relative to each other such that a protector sheath held at the distal end of the delivery catheter fully covers the medical device held at the distal end of the mandrel, whereas, upon the slider mechanism being longitudinally displaced beyond the first deployment feedback position, the slider mechanism positions the mandrel into an at least partly unsheathed configuration in which the mandrel and the delivery catheter are positioned relative to each other such that the protector sheath no more fully covers the medical device.

Thus, a user of the control handle may receive a haptic deployment feedback when displacing the slider mechanism beyond a position where the IMD is transferred from the configuration in which it is fully covered by the protector sheath to the configuration in which it is no more fully covered by the protector sheath, i.e. where it is at least partly or fully ejected from the protector sheath and may therefore e.g. be anchored within the patient's tissue at an intended implantation location.

According to a further implementation, a predetermined second deployment feedback position corresponds to a position such that, upon the slider mechanism being longitudinally slid from the first deployment feedback position to the second deployment feedback position, the slider mechanism successively positions the mandrel into an anchoring configuration relative to the delivery catheter such that an anchoring mechanism at a distal end of the medical device is ejected from the protector sheath in order to anchor the medical device in cardiac tissue, whereas, upon the slider mechanism being displaced beyond the second deployment feedback position towards the fully undeployed position, the slider mechanism successively positions the mandrel into an unsheathed configuration in which the mandrel and the delivery catheter are positioned relative to each other such that the protector sheath no more covers the medical device.

Thus, the user of the control handle may receive a haptic deployment feedback when displacing the slider mechanism beyond a position where the IMD is transferred from a configuration, in which its anchoring mechanism is not yet activated for example by being ejected from the protector sheath, to a configuration, in which the anchoring mechanism is activated in order to anchor the IMD at the intended implantation location.

According to an implementation, the haptic deployment feedback mechanism comprises at least one ridge and at least one bumper. The ridge is arranged at one of the handle housing and the slider mechanism and the bumper being arranged at the other one of the handle housing and the slider mechanism. The ridge and the bumper are configured such as to induce a retracting force onto the slider mechanism upon a user longitudinally pushing the slider mechanism beyond the at least one deployment feedback position.

By inducing the retracting force onto the slider mechanism upon passing the deployment feedback position, the arrangement including the ridge and the bumper may generate the haptic deployment feedback to the user relating to a specific configuration of the delivery system to be indicated to the user. The retracting force may decelerate the longitudinal displacement of the slider mechanism and/or may require the user to temporarily increase his force acting onto the slider mechanism in order to translate the slider mechanism beyond the deployment feedback position.

According to a further specified implementation, the slider mechanism is guided in the handle housing upon being longitudinally displaced and the deployment feedback mechanism is configured such that the ridge and the bumper are elastically biased towards each other in a lateral direction crossing the longitudinal direction.

For example, the ridge may be a protrusion protruding from the handle housing in a direction towards the slider mechanism. The bumper may be an element protruding from the slider mechanism in a direction towards the handle housing. Thus, the ridge and the bumper may protrude in opposite directions. Both, the protruding direction of the ridge as well as the protruding direction of the bumper may be orthogonal to the longitudinal direction of the handle. At least one of the ridge and the bumper may be implemented such as to be elastically displaceable in a direction against the protruding direction. Accordingly, upon a force being effected onto the ridge and/or bumper in such direction, the respective element may be drawn aside, i.e. may move or deflect away from the other element. Due to such action, the bumper and ridge temporarily induced a higher retracting force acting against the displacement of the slider mechanism relative to the handle housing and may therefore generate the haptic deployment feedback.

According to an implementation, the control handle further comprises a valve arranged in the slider mechanism such as to prevent back-bleeding through a lumen of the delivery catheter.

Such valve may be arranged within the slider mechanism at a location at or adjacent to the lumen of the delivery catheter and may block a passage through which blood coming from such lumen may otherwise flow through the control handle. Accordingly, the valve may prevent that blood entering the delivery catheter at the distal end within the patient is ejected at the proximal end of the delivery catheter, thereby potentially polluting components of the control handle or even being ejected from the control handle.

According to a further specified implementation, the valve is formed by a membrane held in the slider mechanism in a cross-sectional direction and having a through-hole configured for tightly enclosing the mandrel extending through the slider mechanism and through the through-hole of the membrane.

Thus, the valve may seal a passage through the control handle where the mandrel generally extends from a position proximal to the control handle to an extension portion extending through the delivery catheter distal of the control handle. Due to such membrane valve, no blood may flow from inside the delivery catheter in a direction parallel to the mandrel towards locations at a proximal side of the control handle.

The delivery system according to the second additional aspect comprises a control handle according to an implementation of the first additional aspect. Furthermore, the delivery system comprises an elongate delivery catheter mechanically connected to the control handle and a mandrel being controlled by the control handle such as to be displaceable longitudinally relative to the delivery catheter. Therein, the control handle may be configured and may cooperate with the delivery catheter and the mandrel as described further above and further below. Furthermore, the delivery catheter and the mandrel may be configured for implementing various functionalities.

Particularly, according to an implementation, the delivery system may be configured such that, upon the mandrel being displaced relative to the delivery catheter by the deployment distance, the medical device held at a distal end of the delivery catheter is deployed from a protector sheath of the delivery catheter. Furthermore, the delivery system may comprise a tethering member being connected to a distal end of the mandrel and being connected to the medical device, and the delivery system may be configured such that, upon the mandrel being displaced relative to the delivery catheter by a tethering distance additionally to the deployment distance, the tethering member is displaced between a retracted position and an ejected position. In the retracted position, the tethering member pulls the medical device into fixation with an end cup at the distal end of the delivery catheter whereas, in the ejected position, the tethering member releases the medical device from the fixation with the end cup.

According to a further specified implementation, the mandrel at least comprises a bare mandrel portion and a tether hypotube portion being arranged further proximal to the bare mandrel portion. The bare mandrel portion has a first cross-sectional dimension and the tether hypotube portion has a second cross-sectional dimension being larger than the first cross-sectional dimension. The length of the bare mandrel portion corresponds to or is longer than the deployment distance and the length of the tether hypotube portion corresponds to or is longer than the tethering distance.

It shall be noted that possible features and advantages of embodiments of the invention are described herein with respect to various embodiments of a control handle and with respect to various additional aspects and implementations of such control handle. One skilled in the art will recognize that the features may be suitably transferred from one embodiment or implementation to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
- Fig. 1: shows a partially exploded longitudinal-sectional view of a control handle in a deployment system according to an embodiment of the present invention.
- Fig. 2: shows a perspective view onto a portion of the control handle of Fig. 1.
- Figs. 3A-3D: visualise various operational stages upon actuating a slider mechanism in a control handle in longitudinal-sectional views and associated cross-sectional views.
- Fig. 4: shows details of a control handle in a perspective view and an associated cross-sectional view.
- Fig. 5: shows a cross-sectional view of internal details of a haptic deployment feedback mechanism within a control handle.
- Fig. 6: shows a perspective view onto a locking mechanism in a control handle.
- Fig. 7: visualizes internal details of the locking mechanism of Fig. 6.
- Figs. 8A-8C: visualise various operational stages upon actuating a locking mechanism and a mandrel in a control handle in side views.
- Fig. 9: shows a longitudinal-sectional view of internal details of a locking mechanism.
- Fig. 10A-10C: shows cross-sectional and longitudinal-sectional views of internal details of a haptic locker feedback mechanism within a control handle.

The figures are only schematic and not to scale. Same reference signs refer to same or similar features.

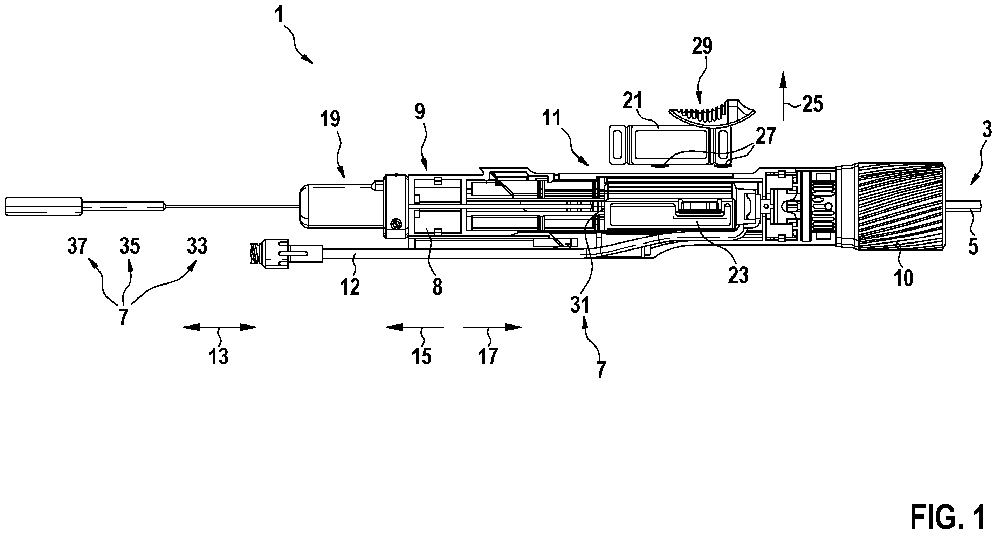

Fig. 1 shows a control handle 1 according to an embodiment of the present invention as well as according to further implementations for controlling functions of a delivery system 3 for implanting a medical device (not shown).

The delivery system 3 comprises an elongate delivery catheter 5 and a mandrel 7 which is displaceable longitudinally relative to the delivery catheter 5 and which also extends through the control handle 1. Therein, a longitudinal positioning of the mandrel 7 controls or influences operations of the delivery system 5 e.g. during surgical procedures in which an IMD, in particular an ILP, held at a distal end of the delivery system 5 is successively deployed, anchored, tested and finally released. While Fig. 1 mainly focuses on visualising details of the control handle 1 and shows only a proximal end portion of the delivery system 3, details of a possible delivery system 3 to be controlled by the control handle 1 and, particularly, details of operation principles of such delivery system 3 have been described in the applicant's prior applications such as WO 2020/043481 A1 and WO 2020/187663 A1.

The control handle 1 comprises a handle housing 9 and a slider mechanism 11. The handle housing 9 is composed of half-shells 8. The slider mechanism 11 may be used for specifically controlling a deployment procedure and/or recapturing procedure at the delivery system as well as selectively activating or deactivating a so-called tether mode at the delivery system 3.

The slider mechanism 11 may be displaced in a longitudinal direction 13 relative to the handle housing 9. Particularly, the slider mechanism 11 may be longitudinally displaced between a fully undeployed position and a fully deployed position. In the fully undeployed position, the slider mechanism 11 is arranged at a most proximal position, i.e. is slid in a proximal direction 15 to a proximal end of a displacement range, whereas, in the fully deployed position, the slider mechanism is arranged at a most distal position, i.e. is slid in a distal direction 17 to a distal end of the displacement range.

The slider mechanism 11 interacts with the mandrel 7 such that, upon displacing the slider mechanism 11 between the fully undeployed position and the fully deployed position, the mandrel 7 is displaced relative to the delivery catheter 5 by a predetermined deployment distance. Furthermore, the slider mechanism 11 is configured such that, when being arranged in the fully undeployed position, the mandrel 7 is fixed relative to the slider mechanism 11 in a manner such that the mandrel 7 may not be displaced relative to the slider mechanism 11 beyond a predetermined stop position due to a mechanical mandrel motion blockage being established. However, when being set to a so-called tether position which may be reached upon displacing the slider mechanism 11 beyond the fully deployed position, the mandrel 7 may be moved in the longitudinal direction relative to the slider mechanism 11 beyond the predetermined stop position due to the mandrel motion blockage being released.

Furthermore, the control handle 1 comprises a locking mechanism 19. The locking mechanism 19 may be specifically used for controlling whether or not the mandrel 7 may be displaced to a position at which the delivery system 3 finally releases the medical device at its distal end from being mechanically connected to the delivery system 3.

The locking mechanism 19 may be switched between a locked configuration and an unlocked configuration. In the locked configuration, the mandrel 7 is fixed relative to the locking mechanism 19 such that it may not be displaced relative to the locking mechanism 19 beyond a predetermined abutment position as a result of a mechanical mandrel abutment blockage being activated. However, upon being switched to the unlocked configuration, the mandrel abutment blockage is released and the mandrel 7 may be moved in the longitudinal direction relative to the locking mechanism 19 beyond the abutment position.

Furthermore, Fig. 1 shows a steering mechanism 10 for steering the delivery catheter 5 and an insertion tube 12 usable for inserting a contrast agent through the delivery catheter 5.

First, details, characteristics and possible advantages of the slider mechanism 11 in the control handle 1 for controlling deployment or retraction of the IMD and for furthermore controlling activation or deactivation of the tether mode in the delivery system 3 will be described with reference to figures 1 to 5. Therein, the details, characteristics and possible advantages are described with reference to an exemplary embodiment or implementation relating to an implantation procedure of a leadless pacemaker.

The implantation procedure of a leadless pacemaker generally requires an implantation catheter that is capable of steering, positioning, and delivery of the implant to a specific location of the heart. A normal implantation procedure to implant a leadless pacemaker in either the right ventricle or atrial appendage of the heart utilizes access to the femoral vein of a patient. An introducer is installed that traverses from the incision site up into the atrium of the heart. Once the introducer is in place, a catheter is required that can safely house the implant and its fixation mechanism and safely protect it from harmful interactions with either the patient's anatomy or the introducer. The catheter is then inserted through the introducer until it exits the introducer in the atrium. Once the catheter's distal end is no longer contained within the introducer, it must navigate either across the tricuspid valve and into the right ventricle or navigate directly up from the atrium and over to the atrial appendage. Once the distal end of the catheter is in the chosen deployment location, the implant is deployed into the heart wall tissue.

Once the implant has been deployed, tests including electrical and fixation measurements are performed by the user to confirm that an implantation site is suitable prior to release of the implant. If any electrical or fixation testing results are shown to be unacceptable, the implant is recaptured, repositioned, and implanted in a new location. Once the electrical and fixation testing measurements are found to be acceptable, the user shall then release the implant from its tether that is connected to the implantation catheter. After the implant has successfully been released, the tooling is then safely removed from the patient anatomy.

The control handle 1 described herein may serve as an implantation catheter's deployment actuator mechanism that allows the user to both deploy the implant, as well as seamlessly and controllably enter into the tether mode which is used to assess the implant's fixation to the heart wall. If the user has determined through measurements that the implantation site is not acceptable, the novel mechanism detailed by this disclosure shall allow the user to safely and reliably exit out of tether mode and recapture the implant back into a catheter's protector cup so that the implant can be repositioned, deployed, and ultimately released at a newly chosen location.

A previous solution in the field consists of using an actuator to retract the outer catheter and protector cup proximally away from the implant in order to deploy the implant into the heart wall. The actuator is thus retracted proximally in order to deploy the implant, and advanced distally in order to recapture the implant. The deployment actuator only controls the deployment process for the implant and is completely decoupled from toggling the tether mode into being active or inactive. This solution uses a separate actuator to control the tether mode which is used to lock and unlock the tether from being movable (i.e. making the tether mode inactive for Lock and active for Unlock). Furthermore, this solution utilizes a long flexible suture as a means for tethering to the implantation catheter.

Furthermore, this tether lock/unlock mechanism is essentially just a stopcock that the suture runs inside of. When the stopcock is locked via the handle actuator, it clamps/cinches down on the suture line inside of it and prevents movement of the suture either distally or proximally. When it is actuated to the unlocked position, the stopcock releases its cinch on the suture. Furthermore, the stopcock has no valve inside or means to prevent back bleeding when it's in the open/unlocked position. Thus, once this mechanism becomes unlocked and tether mode becomes engaged, it is essentially an open tube that is connected to the catheter's wet pathway in which blood can slowly drip backwards out of the catheter's proximal end and around the tether itself. This means that any flushing of the catheter while it is in tether mode is not advised as the flushed fluid medium will just exit out of the proximal end of the catheter and onto the patient or user.

Another currently marketed solution has a different approach consisting of two flexible release pins to maintain a tether and a handle that deploys the implant by screwing it into the heart wall.

Another approach uses separate actuators to deploy the implant and to toggle the tether mode between active/inactive. The multiple actuators result in decreased usability for users as they have more controls to actuate and steps to perform and remember throughout the implantation procedure. Furthermore, since the actuators are decoupled, there presents an opportunity for the user to begin the recapture process accidently without having inactivated the tether mode by locking the tether actuator. This can result in complications during recapture and potentially damage to the implantation catheter during the recapture process (e.g. if the user failed to lock the tether prior to attempting the recapture process).

Furthermore, the stopcock has no valve inside or means to prevent back bleeding. Thus, once this mechanism becomes unlocked and tether mode becomes engaged, it is essentially an open tube that is connected to the catheter's wet pathway in which blood can slowly drip backwards out of the catheter's proximal end. This means that any flushing of the catheter while it is in tether mode is not advised as the flushed fluid medium will just exit out of the proximal end of the catheter and onto the patient/user.

The suture is also known to impart excessive friction forces on both the user and the implant during removal, which could potentially result in the user accidently dislodging the implant's fixation mechanism from the heart wall if too much force is accidently imparted by the user during release and removal of the suture. The suture can also increase the risk of clotting or become tangled during use. Finally, the suture has no fluoroscopic visibility.

In view of such conventional approaches, it was an object to design the control handle 1 such as to be a singular actuator and handle mechanism that allows a user a superior means to safely and reliably both deploy a leadless pacer implant and automatically transition into tether mode, and also exit from tether mode and recapture the leadless pacer implant so that it can be repositioned to a new location, redeployed, and eventually fully released from the catheter.

The control handle 1 comprises the slider mechanism 11. The slider mechanism 11 is configured for automatically transitioning from a deployment mode to a tether mode. Specifically, the slider mechanism 11 is configured such that, when the slider mechanism is displaced from the fully undeployed position to the fully deployed position and then all forces applied by the user acting onto the slider mechanism are released, the slider mechanism automatically moves into a tether position in which the mandrel motion blockage is released.

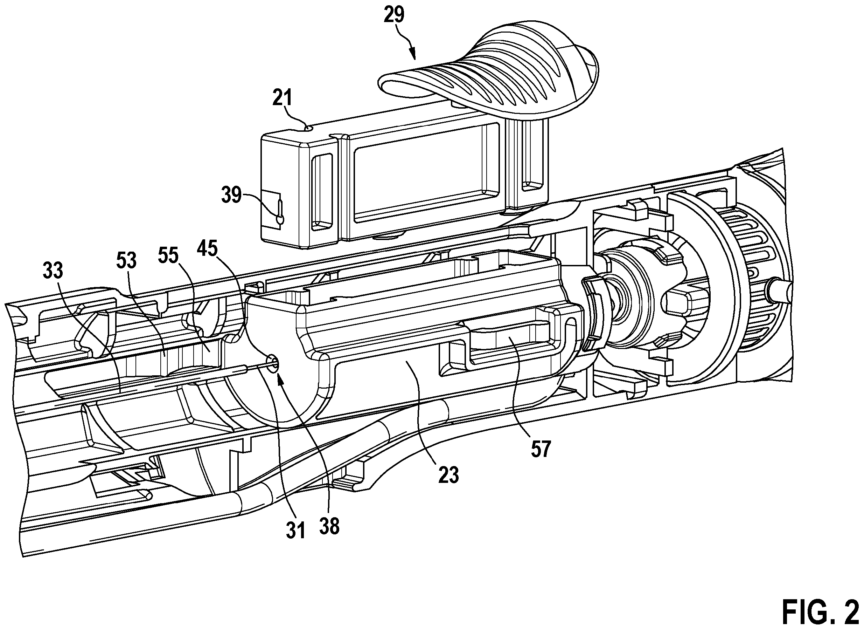

For such configuration and as shown in Fig. 1 and in more detail in Figs. 2 to 5, the slider mechanism 11 comprises a slider body 21 and a slider housing 23.The slider body 21 is arranged at or in the slider housing 23 such as to be elastically biased in a lateral direction 25 away from the slider housing 23. For such purpose, some slider springs 27 are arranged between the slider body 21 and the slider housing 23. Furthermore, at a side opposite to the side of the slider housing 23, the slider body 21 comprises a textured actuation structure 29 via which the user may displace the slider mechanism 11 in the longitudinal direction 13, i.e. in the proximal direction 15 for a deployment procedure or in the distal direction 17 for a recapturing procedure, and via which the user may additionally exert a pressure onto the slider body 21 against elastic bias in the lateral direction 25 or release such pressure.

Therein, the slider body 21 and the slider housing 23 are configured such that, as long as the slider mechanism 11 is positioned in the fully undeployed position or somewhere between the fully undeployed position and the fully deployed position, the slider body 21 is kept fixed to the slider housing 23 in a pulled-down configuration. However, upon arriving at the fully deployed position, such fixation is automatically released such that the slider body 21 automatically displaces in the lateral direction 25 away from the slider housing 23 into a pulled-up configuration due to the elastic biasing forces. As a result of such lateral displacement of the slider body 21, the mandrel motion blockage is automatically released and the mandrel 7 may therefore be longitudinally displaced beyond the predetermined stop position.

Therein, the predetermined stop position is determined due to the mandrel 7 having different portions, the portions differing from each other with respect to their cross-sectional dimensions, i.e. with respect to their diameter. In the example shown in Fig. 1, the mandrel 7 comprises a bare mandrel portion 31. Further proximal to such bare mandrel portion 31, the mandrel 7 comprises a tether hypotube portion 33 having a larger diameter than the bare mandrel portion 31. Further proximal to such tether hypotube portion 33, the mandrel 7 further comprises a release hypotube portion 35 having an even larger diameter than the tether hypotube portion 33. At a most proximal end, the mandrel 7 comprises a tether actuator 37 having a larger diameter than the release hypotube portion 35.

The individual lengths of each of the portions 31, 33, 35 of the mandrel 7 are set such that upon displacing the mandrel 7 longitudinally relative to the delivery catheter 5, various procedures or modes may be induced at the distal end of the delivery system 3 such as a deployment/recapturing procedure/mode, a tether procedure/mode and a release procedure/mode. Therein, the different diameters of the mandrel portions 31, 33, 35 may be used for implementing blockages for preventing that the user unintentionally displaces the mandrel 7 beyond a predetermined stop position, thereby unintentionally switching from one of such procedure/mode to another one procedure/mode.

Described in more details with reference to Figs. 1 to 3, the design solution of the control handle 1 comprises the deployment slider mechanism 11 with the slider body 21 having a haptic surface that is intended for a user to advance or retract the slider using either their thumb or finger. The slider body 21 has a keyed cutout running entirely through its interior that is referred to as the tether control groove 38. Such keyed cutout is formed by a through-hole 39 extending in the longitudinal direction such that the mandrel 7 may extend longitudinally through such through-hole 39. The through-hole 39 comprises a keyed contour with a first section 41 having a smaller cross-sectional dimension than a second section 43. The slider housing 23 as another through-hole 45. The through-hole 45 in the slider housing 23 and the through-hole 39 in the slider body 21 are aligned with each other such that the mandrel 7 may extend longitudinally through both through-holes 39, 45. Therein, depending on the lateral positioning of the slider body 21 relative to the slider housing 23, the smaller first section 41 or the larger second section 43 of the through-hole 39 in the slider body 21 is aligned with the through-hole 45 in the slider housing 23, the latter having a same or larger diameter than the larger second section 43 in the through-hole 39 of the slider body 21.

Accordingly, when the slider body 21 is in its pulled-down configuration, the mandrel 7 extends through the smaller first section 41 such that only its bare mandrel portion 31 with its small diameter may extend through the through-holes 39, 45. Accordingly, in such pulled-down configuration, the mandrel 7 may not be displaced beyond the predetermined stop position at which the tether hypotube portion 33 with its larger diameter would have to be inserted into the through-holes 39, 45. However, when the slider body 21 is arranged in its pulled-up configuration, the mandrel 7 extends through the larger second section 43 of the through-hole 39 such that also the larger tether hypotube portion 33 may extend and may displaced through the through-holes 39, 45.

Explained in other words, the slider body 21 may be placed inside of the slider housing 23. Springs 29 located in between the slider body 21 and the slider housing 23 force the slider body 21 to pop out from inside of the slider housing 23 unless the slider body 21 is being depressed. Furthermore, a geometry inside of the left and right halves 8 of the handle housings 9 keep the slider housing 23 from rotating or moving in any direction except axially in a distal or proximal direction 15, 17. The left and right handle housing geometry also controls the position (height) of the slider body 21 from within the slider housing 23. The slider body 21 will always be in the depressed position unless the slider mechanism 11 (i.e. slider body 21 and slider housing 23) has been advanced to its fully deployed position, i.e. its most forward (distal) position. Once it has been advanced to the distal position, the slider body 21 can pop upwards, which allows for the larger second section 43 in the through-hole 39 of the tether control groove 38 to be aligned with the proximal through-hole 45 on the slider housing 23.

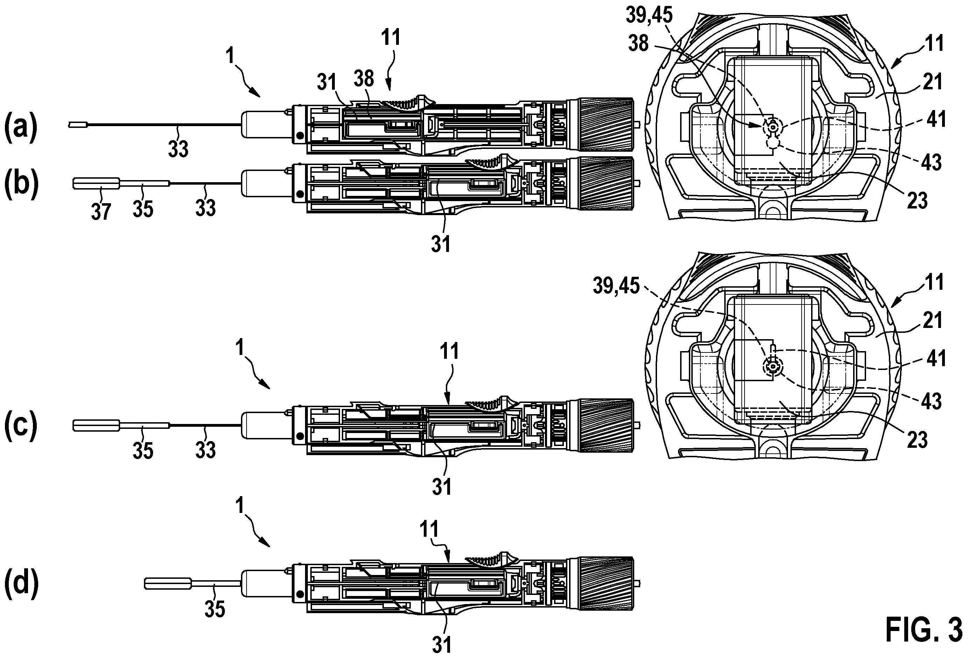

Accordingly, as can be seen in Figure 3, while the slider body 21 is in the depressed, pulled-down configuration (in both fully undeployed through fully deployed position as i.e. proximal through distal positions), the tether hypotube portion 33 is unable to be advanced into the tether control groove 38 in the slider mechanism 11 (see particularly the cross-sectional view in Fig. 3A/B). However, upon the slider body 21 being in the undepressed pulled-up configuration (which only occurs with the slider mechanism 11 being slid to or beyond to its distal, fully deployed position), the tether hypotube portion 33 may be advanced into the tether control groove 38 in the slider mechanism 11 and thus the tether mode may be enabled.

Accordingly, once the larger second section 43 of the through-hole 39 of the slider body 21 of the tether control groove 38 has been aligned with the through-hole 45 of the slider housing 23, the tether hypotube portion 33, which is fixedly attached to the mandrel 7, may now be advanced forward through the slider mechanism 11. This is because the tether hypotube portion 33 has a larger geometry than the bare mandrel portion 31. When the slider body 21 is in the depressed, pulled-down configuration, the keyed inner geometry of the tether control groove 38 thus prevents the tether hypotube portion 33 from being advanceable through it. Once the proximal tether assembly (i.e. tether hypotube portion 33 / bare mandrel portion 31) has been advanced distally through the slider mechanism 11, the tether cable assembly at the distal end of the delivery catheter 5 will have been deployed by the same amount, and thus the tether mode will be in the active state.

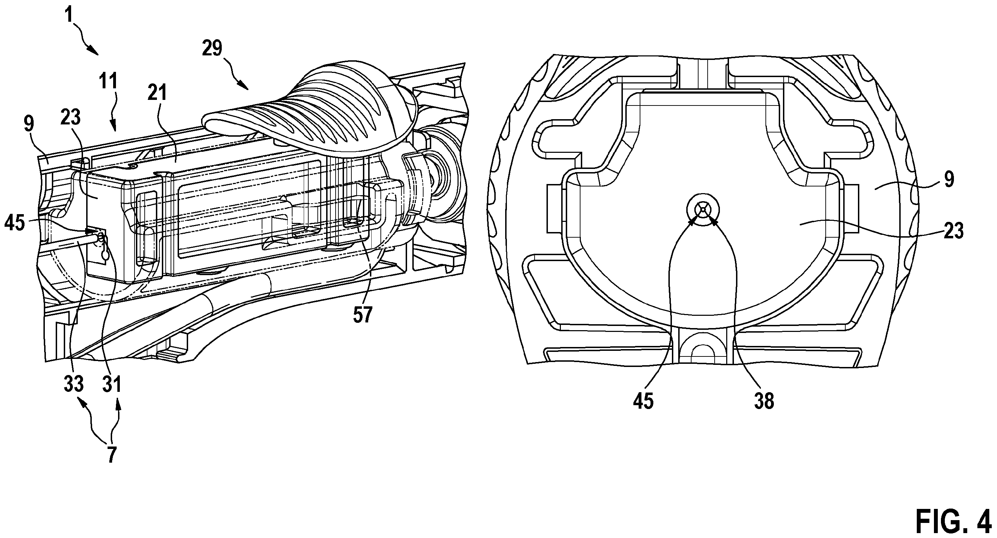

Figure 4 further illustrates how the keyed geometry of the deployment slider either prevents or allows movement of the proximal tether assembly depending on if the slider body 21 has popped up (slider is undepressed) or is in the depressed position.

Fig. 3 also illustrates different stages that the control handle 1 may assume. In a first stage, as shown in Fig. 3A, the slider mechanism 11 is in its retracted state, i.e. in its fully undeployed position. The slider body 21 is depressed such that the tether may not be advanced, i.e. the tether hypotube portion 33 of the mandrel 7 may not be advanced beyond the predetermined stop position and the mandrel motion blockage is active. In a second stage, as shown in Fig. 3B, the slider mechanism 11 is slid to its advanced state, i.e. in its fully deployed position. The slider body 21 is still depressed by the user. Accordingly, the tether may still not be advanced. In a third stage, as shown in Fig. 3C, the slider mechanism 11 is still in the advanced state, i.e. at a position longitudinally corresponding to the fully deployed position, but the user has removed their hand from the slider body 21, i.e. has released the lateral pressure onto the slider body 21. Accordingly, the slider body 21 is allowed to pop-up into its pulled-up configuration. Thus, the tether mode is engaged and the tether may be further advanced, i.e. the tether hypotube portion 33 of the mandrel 7 may be advanced beyond the predetermined stop position and the mandrel motion blockage is deactivated. In a fourth stage, as shown in Fig. 3D, the slider mechanism is still in the advanced state and the slider body 21 is in its pulled-up configuration while the tether having been maximally advanced. In such maximally advanced configuration, further advancement of the mandrel 7 is blocked by the locking mechanism 19, as will be described further below.

This slider mechanism 11 is also designed to facilitate safe recapture of the medical device forming the implant. In order to safely recapture the implant, the tether must be pulled completely proximal (backwards) so that the tether cable slack has been removed and is taut and the implant's hitch has been pulled back into the catheter's alignment cup. If this was not done, and tether cable slack was still present at the distal end of the catheter, the implant's hitch could get caught on the catheter's protector cup and either damage/kink the protector cup, or prevent the catheter from resheathing the implant entirely. To prevent this from happening, the user is mandated to pull the tether all the way back proximally until the tether hypotube is proximal of the deployment slider. This results in the hitch safely being positioned inside of the catheter's alignment cup. If the tether hypotube is at all forward inside the deployment slider (resulting in slack of the tether cable at the distal end of the catheter), the user is unable to depress the deployment slider downwards and retract the deployment slider assembly proximally. This is because the deployment slider has been designed to not be able to be fully depressed if the tether hypotube portion is still at all inside of it. If the deployment slider is not fully depressed, then it can't be retracted and thus the implant cannot be resheathed. Thus, this mechanism has a built-in safety feature to prevent users from inadvertently deactivating the tether mode and unsafely recapturing the implant if they have not put the implant in a safe position for recapture.

Due to all of these design characteristics and considerations, this novel mechanism can automatically transition between a deployed and tethered state and then back into a non-tether mode/ resheathable state for the implant, all by using a single actuator to control these modes.

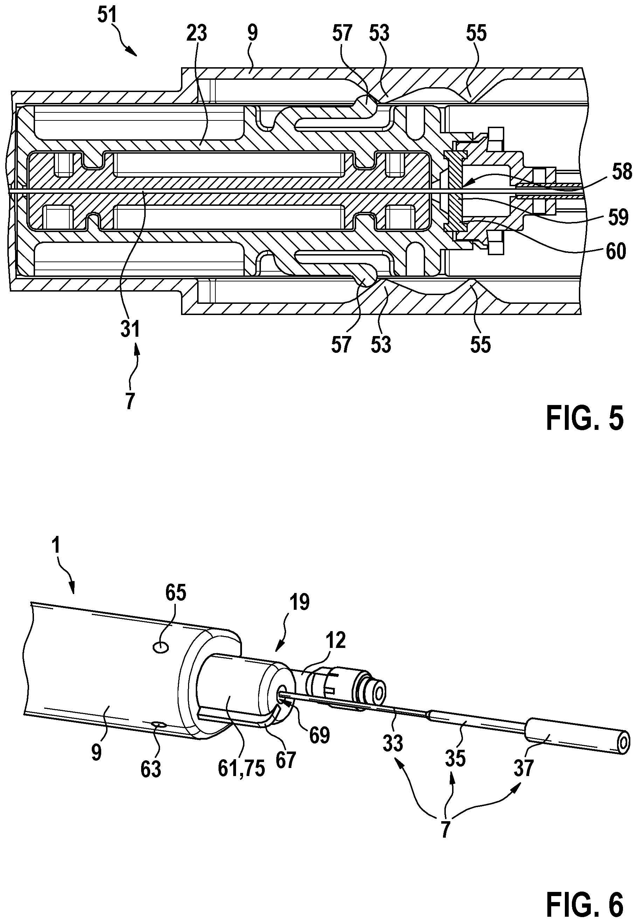

Next, another possible feature of the control handle 1 relating to a haptic deployment feedback mechanism 51 will be described is with reference to Figs. 2 and 5. Such deployment feedback mechanism 51 is configured for generating a haptic deployment feedback which may be sensed by a user of the control handle 1 upon the slider mechanism 11 being displaced beyond one or more predetermined deployment feedback positions along its travel path between the fully undeployed position and the fully deployed position.

Therein, a first deployment feedback position may be at a location indicating a position at which the slider mechanism 11 begins to push the mandrel 7 such that the medical device fixed to the mandrel 7 at its distal end is ejected from a protector sheath. Such first deployment feedback position may also be referred to as proximal deployment control position. Accordingly, until sensing the haptic feedback at the first deployment feedback position, the user knows that the slider mechanism 11 positions the mandrel 7 into a sheathed configuration in which the mandrel 7 and the delivery catheter 5 are positioned relative to each other such that the protector sheath held at the distal end of the delivery catheter fully covers the medical device held at the distal end of the mandrel 7, whereas, upon the slider mechanism 11 being longitudinally displaced beyond such first deployment feedback position, the slider mechanism 11 positions the mandrel 7 into an at least partly unsheathed configuration in which the mandrel 7 and the delivery catheter 5 are positioned relative to each other such that the protector sheath no more fully covers the medical device. For example, in such unsheathed configuration, anchoring tines of the medical device may be deployed.

A second deployment feedback position may be at a location such as to indicate to the user that an anchoring procedure is started upon further sliding the slider mechanism 11 towards the fully undeployed position. Such second deployment feedback position may also be referred to as a distal deployment control position or indicating a haptic pause for implant fixation deployment. Accordingly, upon sensing the haptic feedback at the second deployment feedback position, the user knows that the slider mechanism 11 successively positions the mandrel 7 into an anchoring configuration relative to the delivery catheter 5 such that an anchoring mechanism at the distal end of the medical device is ejected from the protector sheath in order to anchor the medical device in cardiac tissue, whereas, upon the slider mechanism 11 being displaced beyond the second deployment feedback position towards the fully undeployed position, the slider mechanism 11 successively positions the mandrel 7 into an unsheathed configuration in which the mandrel 7 and the delivery catheter 5 are positioned relative to each other such that the protector sheath no more covers the medical device.

Therein, the haptic deployment feedback mechanism 51 comprises at least one ridge 53, 55 and at least one bumper 57. For example, a first ridge 53 may protrude in an inward direction from the handle housing 9 towards the slider housing 23 at a longitudinal position corresponding to the first haptic feedback position and therefore serving as a proximal deployment control ridge. A second ridge 55 may protrude in the inward direction at a longitudinal position corresponding to the second haptic feedback position and therefore serving as a distal deployment control ridge. A bumper 57 may be provided at the slider housing 23 and may protrude in an outward direction towards the handle housing 9.

As shown in Fig. 5, the deployment feedback mechanism 51 may comprise two first ridges 53 and two second ridges 55 opposing each other and protruding inwardly, respectively, at the handle housing 9 and may further comprise two bumpers 57 protruding at the slider housing 23 outwardly. Accordingly, the ridges 53, 55 and the bumpers 57 may slide along each other upon the slider mechanism 11 being longitudinally displaced and induce a retracting force onto the slider mechanism 11 upon the user longitudinally pushing the slider mechanism 11 beyond one of the deployment feedback positions. Therein, the ridges 53, 55 and the bumpers 57 are elastically biased towards each other in the lateral direction 25.

In other words, the control handle 1 preferably includes features to control the position of the deployment slider assembly and provide haptic feedback to the user as the various states are made active or inactive. The proximal deployment control ridge (located on the left and right handle housings and seen in Figure 5) is used to lock the deployment slider assembly in a proximally sheathed position unless the user exerts enough force to advance the deployment slider assembly past the proximal control ridge. The deployment slider housing has flexible bumpers that provide a friction-based interaction with the deployment control ridges that result in a haptic response to the user when the bumpers of the deployment slider housing are advanced against the ridges. These bumpers can also be seen in Figure 5. The user must exert a certain amount of forward force to move the deployment slider assembly, and therefore the bumpers of the deployment slider housing, past each deployment control ridge. Once the deployment slider assembly has been advanced past the proximal deployment control ridge, it is important that the user does not rapidly continue to advance the implant forward - otherwise the implant's fixation mechanism and implant itself could cause a perforation if they are advanced too quickly. Thus, a second distal deployment control ridge is located at the position that corresponds to the implant's fixation being deployed from the catheter's protector cup. This distal control ridge provides a haptic pause for the user to both let them know that the tines have been deployed, and also to proceed from that point forward with caution while they continue the deployment process. The control ridges can be seen in Figs. 2 and 5.

Furthermore, as shown in Fig. 5, the control handle 1 further comprises a valve 59 in the slider mechanism 11 such as to prevent back-bleeding through a lumen of the delivery catheter 5. The valve 59 is formed by a membrane 60 held in the slider mechanism 11 in a cross-sectional direction and having a through-hole 58 configured for tighly enclosing the mandrel 7 extending through the slider mechanism 11 and through the through-hole 58 of the membrane 60. The valve 59 is assembled into the slider housing 23 and is used to prevent back-bleeding through the tether catheter inner diameter and around the tether, and potentially into both the slider mechanism 11 and control handle 1.

The design solution including the slider mechanism 11 described herein incorporates, inter-alia, the following key features:
1.) A singular actuator for both implant deployment and automatically transitioning the tether mode into an active state.
2.) A singular actuator for both implant recapture and automatically transitioning the tether mode into an inactive state.
3.) A deployment slider with a keyed inner geometry that controls the position of the proximal tether assembly (tether hypotube and tether mandrel) and prevents the tether from being advanceable and activating the tether mode if the implant is not fully deployed.
4.) This mechanism also prevents the user from fully depressing the deployment slider and retracting both it and the tether simultaneously for implant resheathing if the tether has first not been fully retracted.
5.) A handle housing geometry that controls the position (height) and orientation of the deployment slider (and keyed inner geometry) relative to the deployment slider housing.
6.) A tether with multiple different outer diameters, with the smaller diameter fitting in the smaller keyed geometry of the deployment slider and the larger outer hypotube which can only be advanced through the largest hole on the deployment slider keyed inner geometry.
7.) Haptic bumpers on the deployment slider housing that provide users with a haptic sensation as they are transitioning through the various stages of the implant deployment process.
8.) Ridges on the left and right handle housings that interact with the bumpers on the deployment slider housing that both lock the deployment slider assembly in the proximal sheathed position, and also provide the user with a haptic pause once the implant's fixation mechanism has been deployed.
9.) A centrally located valve within the deployment slider housing to prevent back-bleeding or air ingress, at all times, back through the tether catheter lumen.

A core concept associated with the control handle 1 including the slider mechanism 11 centers on the realization of a singular actuator that is used to both deploy the implant in a safe and controlled manner, and then once the implant is deployed, to automatically unlock the tether so that the tether mode is active and the tether can become advanceable. Furthermore, this core concept works in the same manner to automatically lock the tether, once the tether has been fully retracted, so that the implant can be safely recaptured using the same actuator. If for whatever reason the tether has not been fully retracted prior to beginning the implant recapture process, the deployment slider mechanism will not allow the user to recapture the implant until the catheter has been put in a state for safe and reliable implant resheathing.

Such approach differs from other market designs which uses two independent actuators to 1.) deploy the implant, and 2.) lock and unlock the tether so that the tether mode can become active or inactive, rather than the singular actuator that does both as described by the present approach described by this disclosure. It also differs from another current market design due to the inclusion of haptic feedback during the deployment process. Furthermore, currently marketed products do not include any haptic feedback features or technology within the implantation catheter. It also again differs from the other market designs which does not feature an inner valve to prevent back-bleeding at all times during use, back around the tether and through the tether catheter ID/lumen. When the currently marketed product enters into tether mode and unlocks the tether, back-bleeding through the tether catheter lumen can occur. In the approach presented herein, the inclusion of a valve (which is active and sealing at all times) prevents back bleeding through the tether catheter inner diameter (ID) at all times. This also means that fluid mediums can be flushed through the tether catheter at any point, even when the catheter is in tether mode.

The presented control handle is specifically designed to incorporate and utilize a novel tether mechanism as described in the applicant's applications filed earlier to maintain a connection to the implant until the user is sure that the implantation site location is acceptable, at which point they can safely release the tether and remove the catheter tooling.

Finally and briefly summarised in alternative wording, the control handle including the slider mechanism described herein includes a novel mechanism for a leadless pacemaker implantation catheter that allows a user to control with a single actuator both the deployment of the implant, and also when the secondary implantation state, known as tether mode, becomes active or inactive. This novel design provides a superior means of controlling the deployment process of a leadless pacemaker, while also allowing the user to seamlessly transition into a state known as tether mode, all with a singular actuator and internal mechanism in the implantation catheter. This novel design provides a superior means for a user to safely and reliably toggle between the controlled deployment of an implant, followed by the seamless transition into the tether mode (this mode is used to assess the implant's fixation, free of any bias from the catheter), and then either to recapture of the implant if necessary or progress to the implant's release from its tethered connection, if the location and fixation are determined to be appropriate. To accomplish this, the novel mechanism uses an advanceable and retractable slider that has an internal mechanism that controls what size geometry can pass through it, depending on what position the slider is in. This mechanism, in conjunction with a tether that incorporates different sizes of outer diameter geometry along its proximal length in the handle, is able to thus determine if the handle's tether mode is active or inactive depending on the position of the deployment slider actuator relative to the implantation catheter handle. This novel leadless pacemaker deployment and tether control mechanism leverages a novel tether mechanism as detailed within earlier invention disclosures as a way to establish a tethered connection between the implant and the catheter. However, the scope of the disclosure presented herein is specific to the deployment actuator and tether control mechanism that is located in the implantation catheter's handle.

Next, details, characteristics and possible advantages of the locking mechanism 19 in the control handle 1 for selectively controlling activation or deactivation of the release mode in the delivery system 3 will be described with reference to figures 6 to 10. Therein, the details, characteristics and possible advantages are again described with reference to an exemplary embodiment or implementation relating to an implantation procedure of a leadless pacemaker.

The control handle 1 described herein may be provided with a specific design of an implantation catheter's locking mechanism 19 serving as a release actuator mechanism that allows the user to begin the release process of the implant from the catheter. If the release actuator has not been actuated, the user will be prevented from releasing the implant from the catheter. Thus, in an implantation procedure, unintentional releasing the implant from the catheter may be reliably prevented for example during deploying the implant from the catheter, anchoring the implant and conducting various tests while the delivery system is in its tether mode, whereas, after such steps, the locking mechanism may be deactivated such as intentional releasing the implant may be conducted by suitably longitudinally displacing the mandrel 7 relative to the delivery catheter 5.

A previous solution in the field consists of using a pair of scissors to cut the tether (which is a suture) of a leadless pacemaker catheter in order to begin the release process of the implant from its tether (suture) to the catheter. Given the pair of scissors are a separate tool, their implant catheter does not consist of a dedicated actuator to begin the release process.

In an alternative approach, the tether consists of a suture line that runs from the proximal end outside of the handle, all the way through the catheter and around the implant's hitch, and then back through the catheter and terminating outside the proximal end of the handle. Both ends of the suture line are fixed to each other and cannot be separated. In order to release the implant from its tether, a user must cut the suture line with a pair of scissors and then retract the suture line fully out of the catheter in order to release the implant from the tether to the catheter.

There is generally no means to begin the release process unless the user has a pair of scissors or a blade on hand to cut the suture line. There are no built-in features, actuators, or mechanisms to cut the suture line present within the handle. Thus, in order to complete an implantation procedure, additional tooling is required that is not included within the catheter system.

In view of such conventional approaches, it was an object to design the control handle 1 such as to prevent a user from being able to release an implant from its tether to the implantation catheter unless the user actuates the mechanism. Preferably, the mechanism should provide the user with clear haptic feedback that the mechanism has been actuated once the user performs the action. The mechanism must remain in a locked state at all times unless actuated by the user.

The control handle 1 comprises the locking mechanism 19 as shown in Figs. 6 to 10. Therein, the locking mechanism 19 may be switched between the locked configuration and the unlocked configuration by rotating an unlock actuation member 61 relative to the handle housing 9. The unlock actuation member 61 may also be referred to as release actuator. A rotation axis of the unlock actuation member 61 is generally aligned with the longitudinal axis of the control handle 1. Preferably, the locking mechanism 19 is configured for being switched between the locked configuration and the unlocked configuration by screwing the unlock actuation member 61 in a clockwise direction along a left-handed thread. The locked configuration may indicated for example by a "lock" icon 63 shown on the handle housing 9, whereas the unlocked configuration may be indicated for example by an "unlock" icon 65. A rotation status of the locking mechanism 19 may be indicated by visual means such as a coloured protrusion 67 provided on the unlock actuation member 61.

For such configuration, the locking mechanism 19 comprises a through-passage 69 for accommodating the mandrel 7. Therein, lateral limiting elements 71 of the locking mechanism 19 enclose the through-passage 69 from opposite sides. The locking mechanism 19 is specifically configured such that, in the locked configuration, the lateral limiting elements 71 are pushed towards each other to a greater extent than in the unlocked configuration such that, in the locked configuration, lateral dimensions of the through-passage 69 are smaller than in the unlocked configuration.

For such purpose, the locking mechanism 19 comprises a compression element 75. Such compression element 75 is configured for releasing compression forces radially acting onto the lateral limiting elements 71 upon rotating the unlock actuation member 61 from the locked configuration to the unlocked configuration. In the example presented in the figures, this compression element 75 is formed by the unlock actuation member 61 itself. However, alternatively, the compression element 75 may be a separate element which cooperates with the unlock actuation member 61.

In the example shown in the figures, the lateral limiting elements 71 are formed by an inner member 77 which encloses the through-passage 69. Therein, longitudinal slits 73 extend between elongate portions 79 of the inner member 77 and separate these elongate portions 79 from each other. The elongate portions 79 extend from a common inner member body portion 81 and have a cantilever end 83 at their opposite sides. At the cantilever end 83, the elongate portions 79 of the inner member 77 have a conical outer shape with a slanted outer surface 85. The compression element 75 is formed by an outer member 87 enclosing the inner member 77. This outer members 87 has a conical inner shape at a slanted inner surface 89 opposing the cantilever end 83 of the inner member 77.

The mandrel 7 comprises a tether hypotube portion 33 and a release hypotube portion 35 being arranged further proximal to the tether hypotube portion 33. The tether hypotube portion 33 has a first cross-sectional dimension, i.e. a first diameter, whereas the release hypotube portion 35 has a larger second diameter.

Accordingly, while the locking mechanism 19 being in the locked configuration, the through-passage 69 has a cross-sectional dimension being smaller than the second cross-sectional dimension of the release hypotube portion 35. Therefore, the mandrel 7 may not be introduced with its release hypotube portion 35 into the locking mechanism 19 and is therefore prevented from being displaced relative to the locking mechanism 19 beyond the predetermined abutment position due to the mandrel abutment blockage being activated. Therein, the abutment position is determined by the location of the transition between the smaller tether hypotube portion 33 and the larger release hypotube portion 35.

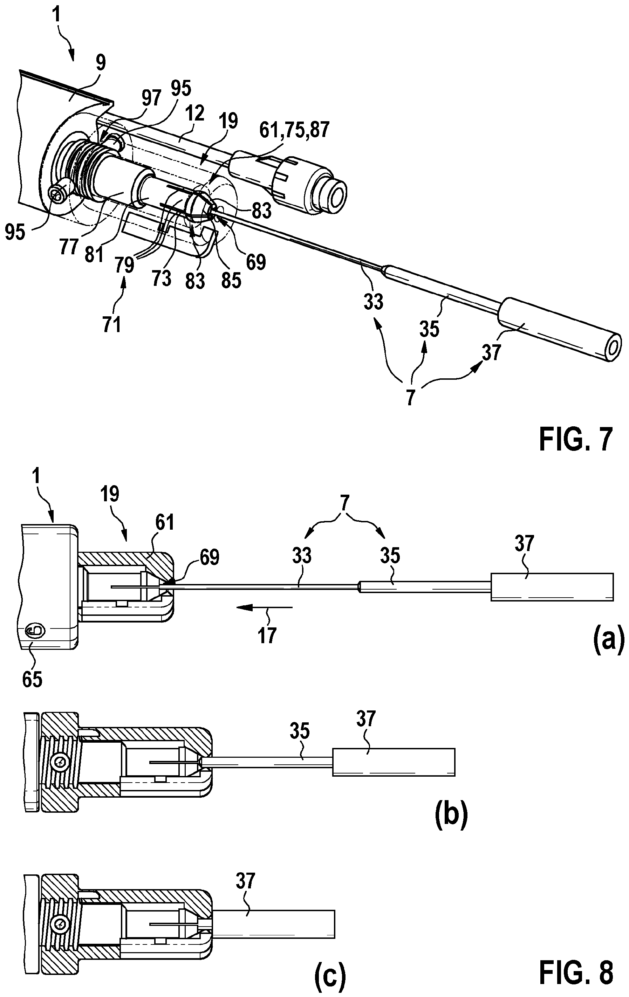

However, upon the locking mechanism 19 being switched to its unlocked configuration, the through-passage 69 obtains a larger cross-sectional dimension which is large enough to accommodate the second cross-sectional dimension of the release hypotube portion 35. Such situation is shown in Fig. 8, wherein sub-figures 8A to 8C visualise that the mandrel 7 may successively displaced in the distal direction 17, i.e. the tether actuator 37 may be pushed towards the control handle 1. Such displacement may be continued beyond a tether mode (as shown in figure 8A) until reaching a release mode (as shown in figures 8B and 8C). Therein, when the locking mechanism 19 being in its unlocked configuration, the mandrel 7 may be pushed in the distal direction 17 until its release hypotube portion 35 is fully pushed through the locking mechanism 19 and the tether actuator 37 abuts to the proximal end of the locking mechanism 19, thereby stopping further displacement of the mandrel 7 (see figure 8C). Due to such continued distal displacement of the mandrel 7, the delivery system is set into a release mode in which the medical device at the distal end of the delivery catheter 5 is released, i.e. a mechanical connection between the medical device and the delivery system 3 is opened.

In other words, the locking mechanism 19 is designed to be used with a tether mechanism with multiple diameters. As seen in the figures 6 to 8, the tether assembly comprises a tether hypotube with a smaller outer diameter (OD) and a release hypotube with a larger OD. In order for the tether to be in a releaseable state, the tether assembly must be advanced distally so that the tether actuator distal face comes to a hard stop against the proximal face of the release actuator. The release actuator prevents the tether from being maximally advanced distally by blocking the release hypotube's larger OD from being advanced through it. A visualization of this and the important positions of the tether relative to the tether actuator can be seen in Fig. 8. Therein, Fig. 8A shows a situation in which the unlock actuation member 61 is switched into the unlocked configuration. Fig. 8(b) visualises that a movement of the catheter distally from this location must be restricted unless the unlock actuation member 61 has been rotated to the unlocked configuration. As shown in Fig. 8C, once the tether has been advanced to its furthest distal position, the tether has been released.

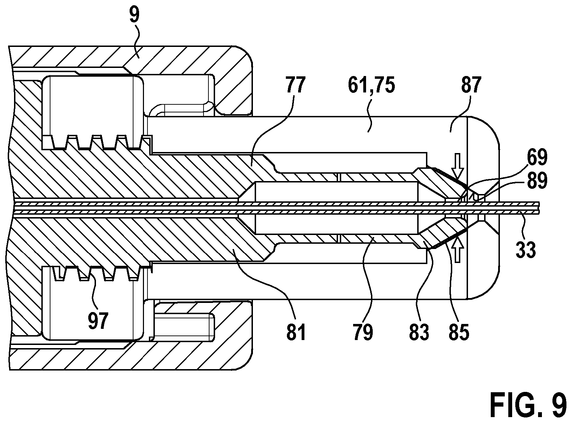

In order to achieve this, the locking mechanism 19 works by the unlock actuation member 61 being screwed inwards (distally) along a thread 97 to a locked position. When the unlock actuation member 61 is advanced in the distal direction 17 via rotation, it radially compresses the slanted outer surfaces 85 at the cantilever ends 83 of the inner elements 77 inward which decreases the inner diameter of the through-passage 69 inside the locking mechanism 19, as shown in Fig. 9. Thus, when the unlock actuation member 61 is in the locked position, it is fully screwed inwards. This results in the inner diameter of the of the through-passage 69 at the locking mechanism 19 being decreased so that it prevents the release hypotube portion 35 from being able to either enter it or be advanced through it.

When the unlock actuation member 61 is in the unlocked position, it is unscrewed which releases the radial compression along the locking mechanism 19, which allows the biased proximal feature inside such locking mechanism 19 to relax back to their original geometry. Once back in their original relaxed geometry, the release hypotube portion 35 may now be advanced distally through this location which will enable the tether to be released. In other words, upon the unlock actuation member 61 being screwed outboards (proximally) to the unlocked position, it is advanced in the proximal direction 15 via rotation, thereby releasing the radial compression onto the cantilever ends 83 of the inner element 77. Thereby, these cantilever ends 83 being elastically biased in an outward lateral direction 25 may relax to a configuration in which the inner diameter of the through-passage 69 is an enlarged such as to enable a passage of the mandrel 7 at its release hypotube portion 35.

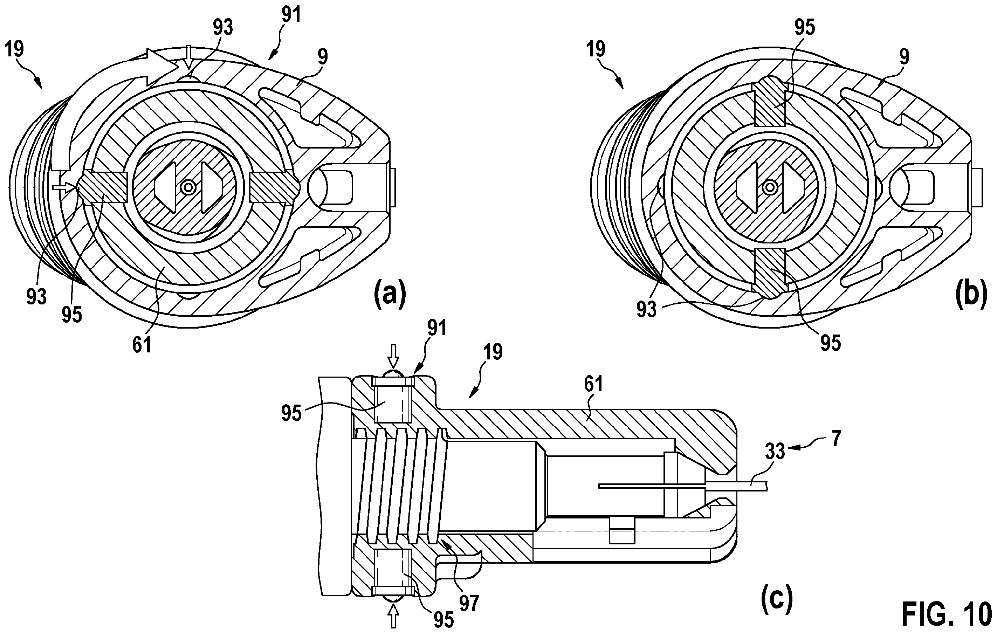

As an additional feature, the control handle 1 further comprises a haptic locker feedback mechanism 91 as visualised in Fig. 10. The haptic locker feedback mechanism 91 is configured for generating a haptic locker feedback to a user of the control handle 1 upon the locking mechanism 19 being displaced relative to the handle housing 9 beyond at least one locker feedback position upon switching from the locked configuration to the unlocked configuration.

For such purpose, the haptic locker feedback mechanism 91 comprises grooves 93 included in the handle housing 9 and spring plungers 95 at the unlock actuation member 61. The grooves 93 and spring plungers 95 are configured such as to induced a retracting force onto the locking mechanism 19 upon a user rotating the unlock actuation member 61 beyond the at least one locker feedback position. For example, there may be grooves 93 at both, the rotation position corresponding to the locked configuration as well as the rotation position corresponding to the unlocked configuration. Accordingly, upon rotating the unlock actuation member 61 between both configurations, its spring plungers 95 may snap into such respective grooves 93, thereby haptically indicating to the user that the locking mechanism 19 has been correctly switched from one configuration to the other.

In other words, another feature of the locking mechanism 19 is that it utilizes spring plungers 95 to create a haptic feedback when it is actuated into either the locked or unlocked states. The spring plungers 95 are embedded into either the release actuator component or the handle component that the release actuator rotates inside of. There are small grooves 93 inside of the handle component to provide spring relief of the spring plungers 95 once the actuator is rotated into either the locked or unlocked position. This relief creates a haptic sensation that the actuator has been rotated into the correct state (either locked or unlocked). The embodiment of the spring plungers 95 being located in the release actuator and being rotated around the handle housing component can be seen in Figure 10.

The design solution including the locking mechanism 19 described herein incorporates, inter-alia, the following key features:
1.) A singular actuator for controlling the release of a tether for a leadless implantation catheter.
2.) An actuator and mechanism that is used to enable release of the implant from the catheter's tether which has been built into the implantation catheter and is not a separate independent tool.
3.) An actuator that when rotated to a locked position, radially compresses an internally located separate component or feature to decrease the ID of a hole or lumen in order to prevent larger geometry features from being advanceable through it.
4.) An actuator that will remain in either a locked or unlocked state unless actuated by a user.
5.) An actuator that when rotated to an unlocked position, radially releases compression upon an internally located separate component or feature that allows that component or feature to relax back into an uncompressed state where its uncompressed state results in a larger ID of the hole or lumen inside of it.
6.) The inclusion of spring plungers and spring relief grooves to provide a haptic response whenever the release actuator is rotated into either the locked or unlocked states.
7.) The inclusion of spring plungers and spring relief grooves to prevent unintended rotation of the actuator unless actuated by a user.
8.) A proximally located tether assembly that incorporates different diameters to restrict advancement of the tether past the release actuator unless the release actuator has been rotated to the unlocked position.
9.) The release actuator mechanism with either clockwise or counter clockwise thread. In our intended embodiment, it is preferred to utilize a CCW thread because it is more difficult for a user to unintentionally actuate the release actuator mechanism to the unlocked position by rotating it away from their body rather than towards their body.

A core concept associated with the present control handle 1 including the locking mechanism 19 centers on the realization of a singular actuator that may be used to prevent the user from unintentionally releasing the implant from its tether to the catheter unless the user is ready to do so, that is capable of controlling the inner diameter of the hole or lumen inside of it. When the actuator is rotated to the locked position, the inner diameter of the inner hole/lumen is decreased. When the actuator is rotated to the unlocked position, the inner diameter relaxes outwards and becomes increased. This release mechanism interfaces with a tether mechanism that consists of multiple outer diameters. When the release actuator is in the locked position, only the smaller diameter of the tether can be advanceable through the release actuator. When the release actuator is in the unlocked position, the larger diameter of the tether can be advanced through the release actuator and the implant can resultingly be released from the tether.

Particularly, the control handle 1 with the locking mechanism 19 differs from the another market design which uses a pair of scissors, not included within the implantation catheter, to sever the suture line which serves as the tether to the implant. By incorporating the release mechanism into the handle, the usability of the leadless pacemaker implantation catheter is greatly enhanced.

The presented control handle 1 is specifically designed to incorporate and utilize a novel tether mechanism as described in the applicant's applications filed earlier to maintain a connection to the implant, as well as interfaces with the novel deployment slider tether control mechanism established by including the slider mechanism as also described herein further above.

Finally and briefly summarised in alternative wording, the control handle including the locking mechanism described herein includes a novel mechanism for a leadless pacemaker implantation catheter that allows a user to control with an actuator, when it is allowable, to release an implant from its tether to the implantation catheter. This novel design utilizes a safety mechanism that prevents the user from beginning the release process of the implant from the catheter unless the actuator is actuated into the unlocked position. Once the actuator is placed into this position, the user can then release the implant from the catheter. This mechanism is intended as a safety precaution to prevent the user from accidently releasing the implant from the catheter unless they are fully sure they are ready to do so. To accomplish this, the novel mechanism uses a rotational screw-based actuator that is rotatable e.g. up to 90 degrees to toggle the actuator between "locked" and "unlocked" configurations. When the actuator is placed in the "locked" configuration, it torques down upon an inner component that becomes compressed inward. When the inner component is compressed inward, the inner component's inner diameter (which the tether mechanism runs through) becomes smaller. With the inner component's inner diameter put in a state with a smaller inner diameter, the tether (which has multiple outer diameters) cannot be fully advanced through this component because the largest size diameter of the tether cannot fit through this handle component while the actuator is engaged in the locked position. The tether mechanism is only releasable after being advanced all the way through this handle component. Once the release actuator is rotated into the "unlocked" position, the torque applied to the inner component is released, and the inner component can return to its natural position. The natural position of the inner component consists of a larger inner diameter. Thus, once the release actuator is in the "unlocked" position, the tether mechanism can now fit through the handle component and the implant can be released from the tether to the catheter. This novel release control mechanism leverages a novel tether mechanism as detailed within earlier invention disclosures as a way to establish a tethered connection between the implant and the catheter.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### LIST OF REFERENCE NUMERALS

- 1: control handle
- 3: delivery system
- 5: delivery catheter
- 7: mandrel
- 8: half-shells
- 9: handle housing
- 10: steering mechanism
- 11: slider mechanism
- 12: insertion tube
- 13: longitudinal direction
- 15: proximal direction
- 17: distal direction
- 19: locking mechanism
- 21: slider body
- 23: slider housing
- 25: lateral direction
- 27: slider spring
- 29: actuation structure
- 31: bare mandrel portion
- 33: tether hypotube portion
- 35: release hypotube portion
- 37: tether actuator
- 38: the control groove
- 39: through-hole through slider body
- 41: first section of through-hole
- 43: second section of through-hole
- 45: through-hole through slider housing
- 51: haptical deployment feedback mechanism
- 53: first ridge
- 55: second ridge
- 57: bumper
- 58: through-hole in valve
- 59: valve
- 60: membrane
- 61: unlock actuation member
- 63: lock icon
- 65: unlock icon
- 67: protrusion
- 69: through-passage
- 71: lateral limiting elements
- 73: slits
- 75: compression element
- 77: inner element
- 79: elongate portion
- 81: common inner member body portion
- 83: cantilever end
- 85: slanted outer surface
- 87: outer member
- 89: slanted inner surface
- 91: haptic locker feedback mechanism
- 93: groove
- 95: spring plunger
- 97: thread

## Claims

1. A control handle (1) for controlling functions of a delivery system (3) for implanting a medical device,
wherein the delivery system (3) comprises
- an elongate delivery catheter (5), and
- a mandrel (7) being displaceable longitudinally relative to the delivery catheter (5);
wherein the control handle (1) comprises:
- a locking mechanism (19), and
- a handle housing (9);
wherein the locking mechanism (19) is configured for being switchable between a locked configuration and an unlocked configuration such that, in the locked configuration, the mandrel (7) is fixed relative to the locking mechanism (19) such that the mandrel (7) is not displaceable relative to the locking mechanism (19) beyond a predetermined abutment position due to a mechanical mandrel abutment blockage, whereas, in the unlocked configuration, the mandrel (7) is movable in the longitudinal direction (13) relative to the locking mechanism (19) beyond the predetermined abutment position due to the mandrel abutment blockage being released.

2. The control handle of claim 1, wherein the locking mechanism (19) is configured for being switched between the locked configuration and the unlocked configuration by rotating an unlock actuation member (61) relative to the handle housing (9).

3. The control handle of claim 2, wherein a rotation axis of the unlock actuation member (61) is aligned with a longitudinal axis of the control handle (1).

4. The control handle of claim 2, wherein the locking mechanism (19) is configured for being switched between the locked configuration and the unlocked configuration by unscrewing the unlock actuation member (61), preferably in a clockwise direction over a CCW/left-handed thread.

5. The control handle of claim 1, wherein the locking mechanism (19) comprises a through-passage (69) for accommodating the mandrel (7), with lateral limiting elements (71) of the locking mechanism (19) enclosing the through-passage (69) from opposite sides, wherein the locking mechanism (19) is configured such that, in the locked configuration, the lateral limiting elements (71) are pushed towards each other more than in the unlocked configuration such that, in the locked configuration, lateral dimensions of the through-passage (69) are smaller than in the unlocked configuration.

6. The control handle of claim 5, wherein the locking mechanism (19) comprises a compression element (75) which, upon rotating an unlock actuation member (61) from the locked configuration to the unlocked configuration, releases compression forces radially acting onto the lateral limiting elements (71).

7. The control handle of claim 5, wherein the lateral limiting elements (71) are formed by an inner member (77) enclosing the through-passage (69) and having longitudinal slits (73) extending between elongate portions (79) of the inner member (77) and furthermore having a conical outer shape at its cantilever end (83), and wherein the compression element (75) is formed by an outer member (87) enclosing the inner member (77) and having a conical inner shape at an inner surface opposing the cantilever end (83) of the inner member (77).

8. The control handle of claim 5, wherein the mandrel (7) comprises a tether hypotube portion (33) and a release hypotube portion (35) being arranged further proximal to the tether hypotube portion (33), wherein the tether hypotube portion (33) has a first cross-sectional dimension and the release hypotube portion (35) has a second cross-sectional dimension being larger than the first cross-sectional dimension, wherein, upon the locking mechanism (19) being in the locked configuration, the through-passage (69) of the locking mechanism (19) has a cross-sectional dimension being smaller than the second cross-sectional dimension of the release hypotube portion (35), whereas, upon the locking mechanism (19) being in the unlocked configuration, the through-passage (69) of the locking mechanism (19) has a cross-sectional dimension being larger than the second cross-sectional dimension of the release hypotube portion (35).

9. The control handle of claim 1, wherein the control handle (1) further comprises a haptic locker feedback mechanism(91), the haptic locker feedback mechanism (91) being configured for generating a haptic locker feedback to a user of the control handle (1) upon the locking mechanism (19) being displaced relative to the handle housing (9) beyond at least one locker feedback position upon switching from the locked configuration to the unlocked configuration.

10. The control handle of claim 9, wherein the haptic locker feedback mechanism (91) comprises at least one groove (93) and at least one spring plunger (95), the groove (93) being arranged at one of the handle housing (9) and the locking mechanism (19) and the spring plunger (95) being arranged at the other one of the handle housing (9) and the locking mechanism (19), and being configured such as to induced a retracting force onto the locking mechanism (19) upon a user rotating the locking mechanism (19) beyond the at least one locker feedback position.

11. A delivery system (3) comprising: a control handle (1) according to claim 1, an elongate delivery catheter (5) mechanically connected to the control handle (1); and a mandrel (7) being controlled by the control handle (1) such as to be displaceable longitudinally relative to the delivery catheter (5).

12. The delivery system of claim 11, wherein the delivery system (3) is configured such that, upon the mandrel (7) being displaced relative to the delivery catheter (5) by a deployment distance, the medical device held at a distal end of the delivery catheter is deployed from a protector sheath of the delivery catheter; and wherein the delivery system (3) further comprises a tethering member being connected to a distal end of the mandrel (7) and being connected to the medical device, wherein the delivery system (3) is configured such that, upon the mandrel (7) being displaced relative to the delivery catheter (5) by a tethering distance additionally to the deployment distance, the tethering member is displaced between a retracted position and an ejected position, wherein, in the retracted position, the tethering member pulls the medical device into fixation with an end cup at the distal end of the delivery catheter whereas, in the ejected position, the tethering member releases the medical device from the fixation with the end cup, and wherein the delivery system (3) is further configured such that, upon the mandrel (7) being displaced relative to the delivery catheter (5) by a release distance additionally to the tethering distance and the deployment distance, the tethering member is displaced between the ejected position and a release position, wherein, in the release position, the tethering member releases the medical device from any connection with the delivery system (3).

13. The delivery system of claim 12, wherein the mandrel (7) comprises a bare mandrel portion (31), a tether hypotube portion (33) being arranged further proximal to the bare mandrel portion (31) and a release hypotube portion (35) being arranged further proximal to the tether hypotube portion (33), wherein the bare mandrel portion (31) has a first cross-sectional dimension, the tether hypotube portion (33) has a second cross-sectional dimension being larger than the first cross-sectional dimension and the release hypotube portion (35) has a third cross-sectional dimension being larger than the second cross-sectional dimension, wherein the length of the bare mandrel portion (31) corresponds to or is longer than the deployment distance, the length of the tether hypotube portion (33) corresponds to or is longer than the tethering distance and the length of the release hypotube portion (35) corresponds to or is longer than the release distance.
